# EUROPEAN PATENT SPECIFICATION

(11) **EP 3 450 546 B1**
(45) Date of publication and mention of the grant of the patent: **13.03.2024**
(21) Application number: 17789739.4
(22) Date of filing: 26.04.2017
(51) Int. Cl.: C12N 5/077

(54) **SKELETAL MUSCLE PRECURSOR CELLS AND PRODUCTION METHOD FOR SKELETAL MUSCLE CELLS**
SKELETTMUSKELVORLÄUFERZELLEN UND HERSTELLUNGSVERFAHREN FÜR SKELETTMUSKELZELLEN
CELLULES PRÉCURSEURS DE MUSCLES SQUELETTIQUES ET PROCÉDÉ DE PRODUCTION DE CELLULES DE MUSCLES SQUELETTIQUES

(30) Priority: 27.04.2016 JP 2016089177; 16.05.2016 JP 2016098037
(43) Date of publication of application: 06.03.2019
(73) Proprietor: Takeda Pharmaceutical Company Limited, Osaka 541-0045 (JP)
(72) Inventor: YOSHIDA, Hiromi, Fujisawa-shi Kanagawa 251-0012 (JP)
(74) Representative: Jones, Nicholas Andrew
(86) International application number: PCT/JP2017/017326
(87) International publication number: WO 2017/188458

(56) References cited:
- WO-A1-2013/073246
- WO-A1-2013/138623
- WO-A1-2015/137419
- WO-A1-2016/168890
- AU-A1- 2014 268 197
- JP-A- 2015 513 896
- MICHAEL SHELTON ET AL: "Derivation and Expansion of PAX7-Positive Muscle Progenitors from Human and Mouse Embryonic Stem Cells", STEM CELL REPORTS, vol. 3, no. 3, 1 September 2014 (2014-09-01), pages 516-529, XP055281322, United States ISSN: 2213-6711, DOI: 10.1016/j.stemcr.2014.07.001
- JEROME CHAL ET AL: "Differentiation of pluripotent stem cells to muscle fiber to model Duchenne muscular dystrophy", NATURE BIOTECHNOLOGY, vol. 33, no. 9, 3 August 2015 (2015-08-03) , pages 962-969, XP055281323, New York ISSN: 1087-0156, DOI: 10.1038/nbt.3297
- BIANCA BORCHIN ET AL: "Derivation and FACS-Mediated Purification of PAX3+/PAX7+ Skeletal Muscle Precursors from Human Pluripotent Stem Cells", STEM CELL REPORTS, vol. 1, no. 6, 1 December 2013 (2013-12-01), pages 620-631, XP055324367, United States ISSN: 2213-6711, DOI: 10.1016/j.stemcr.2013.10.007
- SHELTON,M. ET AL.: 'Derivation and Expansion of PAX7-Positive Muscle Progenitors from Human and Mousse Embryonic Stem Cells' STEM CELL REPORTS vol. 3, 2014, pages 516 - 529, XP055281322
- CHAL, J. ET AL.: 'Differentiation of pluripotent stem cells to muscle fiber to model Duchenne muscular dystrophy' NATURE BIOTECHNOLOGY vol. 33, no. 9, September 2015, pages 962 - 969, XP055281323
- SHI, S. ET AL.: 'BMP antagonists enhance myogenic differentiation and ameliorate the dystrophic phenotype in a DMD mouse model' NEUROBIOLOGY OF DISEASE vol. 41, 2011, pages 353 - 360, XP027580050
- BORCHIN, B. ET AL.: 'Derivation and FACS-Mediated Purification of PAX3+/PAX7+ Skeletal Muscle Precursors from Human Pluripotent Stem Cells' STEM CELL REPORTS vol. 1, no. 6, 17 December 2013, pages 620 - 631, XP055324367
- Xu Xiaoti ET AL: "Human Satellite Cell Transplantation and Regeneration from Diverse Skeletal Muscles", STEM CELL REPORTS, vol. 5, no. 3, 1 September 2015 (2015-09-01), pages 419-434, XP055905626, United States ISSN: 2213-6711, DOI: 10.1016/j.stemcr.2015.07.016

## Description

### [Technical Field]

The present invention relates to a method for producing skeletal muscle cells from pluripotent stem cells. More specifically, the present invention relates to a method for differentiating skeletal muscle precursor cells induced from pluripotent stem cells into skeletal muscle cells.

### [Background Art]

Research is ongoing to induce differentiation of pluripotent stem cells such as iPS cells or ES cells into skeletal muscle cells and apply the skeletal muscle cells to the treatment of muscular dystrophy and the like.

Chal et al. have reported a method for inducing differentiation into skeletal muscle cells by culturing human iPS cells for one day in the presence of a ROCK inhibitor, culturing the obtained cells in a medium containing a GSK3β inhibitor and an ALK2, ALK3 and ALK6 inhibitor, adding FGF2 thereto, and culturing the cells for two days from day 6 in a medium containing HGF, IGF1, FGF2 and an ALK2, ALK3 and ALK6 inhibitor, further for four days in the medium supplemented with IGF1, and subsequently in a medium containing HGF and IGF1 (Non Patent Literature 1).

Shelton et al. have reported a method for inducing differentiation into skeletal muscle cells by culturing human ES cells overnight in the presence of a ROCK inhibitor and culturing the obtained cells for two days in the presence of a GSK3β inhibitor (in combination with GSK3β on days 0 to 7 or by the action of forskolin and FGF2 until day 7 after two days of GSK3β treatment) (Non Patent Literature 2).

WO2013/138623 discloses a medium containing a GSK3β inhibitor and activators of cAMP and FGF (such as TGF, including TGFβ) for use in skeletal muscle differentiation (Patent Document 1).

All of these existing methods causes the growth of induced skeletal muscle precursor cells by the action of growth factors from an early stage while inducing differentiation into skeletal muscle cells. Such methods which involve differentiating pluripotent stem cells into skeletal muscle cells at once always require differentiation culture for 1.5 to 2 months for obtaining skeletal muscle cells and, unfortunately, have the difficulty in achieving stable provision of skeletal muscle cells.

### Citation List

### Non Patent Literature

Non Patent Literature 1:
   Chal et al., Nat Biotechnol. 2015 Sep; 33 (9): 962-9, Differentiation of pluripotent stem cells to muscle fiber to model Duchenne muscular dystrophy.
Non Patent Literature 2:
   Shelton et al., Stem Cell Reports. 2014 Sep 9; 3 (3): 516-29. Derivation and expansion of PAX7-positive muscle progenitors from human and mouse embryonic stem cells.

### Patent Documents

Patent Document 1: WO2013/138623

### Summary of Invention

### Technical Problem

An object of the present invention is to provide a novel method for stably providing skeletal muscle cells derived from pluripotent stem cells. From another viewpoint, an object of the present invention is to provide a method for inducing highly pure skeletal muscle cells from pluripotent stem cells.

### Solution to Problem

The inventors have induced differentiation of pluripotent stem cells into skeletal muscle precursor cells using a GSK3β inhibitor, an ALK (activin-like receptor kinase) inhibitor, and a cAMP inducer. The obtained skeletal muscle precursor cells had a high purity and efficiently differentiated into skeletal muscle cells. Furthermore, the skeletal muscle precursor cells are cryopreservable, and use of this frozen cell preparation enables the induction of stable differentiation into skeletal muscle cells in a short period when needed.

Specifically, the present invention provides the following [1] to [12]:
[1] A method for producing skeletal muscle precursor cells, comprising the steps of:
   (1) culturing pluripotent stem cells in a medium containing a GSK3β inhibitor for 1-4 days;
   (2) culturing the cells obtained in the step (1) in adhesion culture in a medium containing an ALK inhibitor for 1-4 days; and
   (3) culturing the cells obtained in the step (2) in a medium containing a cAMP inducer for 9-26 days.
[2] The method according to [1], wherein the medium in the step (2) contains an AMP kinase inhibitor.
   This medium may contain a BMP signal inhibitor in addition to the AMP kinase inhibitor.
[3] The method according to [1] or [2], wherein the media in the steps (1) to (3) are free from a growth factor.
[4] The method according to any of [1] to [3], wherein the skeletal muscle precursor cells are positive for CD56 and/or PAX7.
[5] The method according to any of [1] to [4], wherein the skeletal muscle precursor cells are positive for CD29 and/or PAX7.
[6] The method according to any of [1] to [5], further comprising the step of purifying CD56-positive cells.
[7] The method according to any of [1] to [6], further comprising the step of purifying CD29-positive cells.
[8] A method for producing myotube cells, comprising
   (1) culturing pluripotent stem cells in adhesion culture in a medium containing a GSK3β inhibitor for 1-4 days;
   (2) culturing the cells obtained in the step (1) in adhesion culture in a medium containing an ALK inhibitor for 1-4 days;
   (3) culturing the cells obtained in the step (2) in adhesion culture in a medium containing a cAMP inducer for 9-26 days to obtain skeletal muscle precursor cells positive for CD56 and/or CD29 and/or PAX7, and optionally purifying CD56-positive cells and/or CD29-positive cells;
   (4) culturing the cells obtained in step (3), or cells obtained by thawing a frozen cell preparation obtained from cells obtained in step (3), in adhesion culture in a medium containing a growth factor for 7-20 days to induce differentiation into myotube cells.
[9] The method according to [8], wherein the medium containing a growth factor of step (4) further contains a cAMP inducer.
[10] The method according to [8], wherein the growth factor of step (4) is any one or two or more members selected from the group consisting of FGF2, FGF1, HGF, EGF and IGF1.
[11] A method for producing skeletal muscle cells, comprising the steps of:
   (1) culturing pluripotent stem cells in adhesion culture in a medium containing a GSK3β inhibitor for 1-4 days;
   (2) culturing the cells obtained in the step (1) in adhesion culture in a medium containing an ALK inhibitor for 1-4 days;
   (3) culturing the cells obtained in the step (2) in adhesion culture in a medium containing a cAMP inducer for 9-26 days to obtain skeletal muscle precursor cells, and optionally purifying CD56-positive cells and/or CD29-positive cells;
   (4) culturing skeletal muscle precursor cells obtained in step (3) and positive for CD56 and/or CD29, in adhesion culture in a medium containing a growth factor; and
   (5) culturing the cells obtained in the step (4) in a medium free from a growth factor.
[12] The method according to [11], wherein the growth factor contained in the medium in the step (4) is any one or two or more members selected from the group consisting of FGF2, FGF1, HGF, EGF and IGF1.

### Advantageous Effects of Invention

The skeletal muscle cell preparation obtained by the methods of the present invention has a high purity and is highly useful for cell drugs or drug screening.

### Brief Description of Drawings

[Figure 1a] Figure 1a shows a scheme of induction of differentiation of iPS cells into the somitic mesoderm (Example 1).
[Figure 1b] Figure 1b shows the expression levels of pan-mesodermal markers BRACHYURY and TBX6 on day 2 of differentiation.
[Figure 1c] Figure 1c shows the expression levels of somitic mesoderm markers PAX3 and PAX7 on day 7 of differentiation.
[Figure 2a] Figure 2a shows a scheme of induction of differentiation of iPS cells into skeletal muscle cells by way of the somitic mesoderm (Example 2).
[Figure 2b] Figure 2b shows the expression levels of skeletal muscle markers MYH3, MYH7, MYH8, ACTA1, MYOG and MYOD1 on week 6 of differentiation.
[Figure 3a] Figure 3a shows a scheme of induction of differentiation of skeletal muscle precursor cells induced from iPS cells into skeletal muscle cells by way of dispersion and plating or dispersion and subsequent cryopreservation (Example 3).
[Figure 3b] Figure 3b shows the expression levels of skeletal muscle cell markers MYH3, MYH7, MYH8, ACTN2, MYOD1, MYF5 and MYOG and a skeletal muscle precursor cell marker PAX7 on week 4 of differentiation.
[Figure 3c] Figure 3c shows the expression levels of a skeletal muscle precursor cell marker PAX7 and skeletal muscle cell markers MYH3, MYH8 and MYOD1 after thawing of cells cryopreserved on week 3 of differentiation and subsequent culture for two weeks with or without the addition of a growth factor and/or FSK.
[Figure 3d] Figure 3d shows results of fluorescently immunostaining cells on week 3 of differentiation from iPS cells and four weeks after dispersion and plating (week 7 of differentiation).
[Figure 3e] Figure 3e shows results of thawing cells dispersed and subsequently cryopreserved on week 3 of differentiation from iPS cells, culturing the cells for two days, then fixing the cells, and detecting the expression of a human satellite cell marker by fluorescent immunostaining.
[Figure 3f] Figure 3f shows results of thawing cells dispersed and subsequently cryopreserved on week 3 of differentiation from iPS cells, and fluorescently immunostaining myotube cells induced from the obtained skeletal muscle precursor cells.
[Figure 3g] Figure 3g shows results of thawing cells dispersed and subsequently cryopreserved on week 3 of differentiation, adding a growth factor and FSK thereto, culturing the cells for two weeks, then culturing the cells for two weeks without the addition of a growth factor and FSK, then fixing the cells, and detecting the expression of a human skeletal muscle cell marker by fluorescent immunostaining.
[Figure 4a] Figure 4a shows a scheme of purification of skeletal muscle precursor cells differentiated by induction from iPS cells, and induction of differentiation of the purified skeletal muscle precursor cells into skeletal muscle cells, or a scheme of cryopreservation of the purified skeletal muscle precursor cells.
[Figure 4b] Figure 4b shows results of fluorescently immunostaining skeletal muscle cells induced from CD56-positive cells purified at the stage of skeletal muscle precursor cells.
[Figure 4c] Figure 4c shows results of culturing CD56-positive cells purified at the stage of skeletal muscle precursor cells for two days, then dispersing and cryopreserving the cells, and fluorescently immunostaining skeletal muscle cells induced by thawing and subsequent plating.
[Figure 5] Figure 5 shows results of fluorescently immunostaining cells under reference culture conditions. The left diagram depicts cells after culture for two weeks in a medium containing a growth factor and FSK, and the right diagram depicts cells on week 4 of culture with the medium replaced with a medium free from a growth factor and FSK from week 2 of culture.
[Figure 6] Figure 6 shows results of fluorescently immunostaining cells cultured in a vessel coated with fibronectin. The left diagram depicts myotube cells on week 2 of culture, and the right diagram depicts skeletal muscle cells on week 4 of culture.
[Figures 7a to 7c] Figure 7 shows results of fluorescently immunostaining cells cultured with different reagents. Figure 7a shows cells cultured using 1 µM A83-01SB instead of SB. Figure 7b shows cells cultured using 1 µM A83-01 and 0.1 µM LDN193189 instead of SB and DM. Figure 7c shows cells cultured using 50 µM dbcAMP instead of FSK.
[Figures 8a to 8d] Figure 8 shows results of fluorescently immunostaining cells cultured with different reagents. Figure 8a shows cells cultured using 0.5 µM BIO instead of CHIR. Figure 8b shows cells cultured using 10 µM SB216763 instead of CHIR. Figure 8c shows cells cultured by starting differentiation in a medium supplemented with 0.5 µM BIO instead of CHIR and using 1 µM A83-01 instead of SB on day 2 of differentiation. Figure 8d shows cells cultured using 1 µM A83-01 and 0.1 µM LDN193189 instead of SB and DM.
[Figures 8e to 8h] Figure 8e shows cells cultured by starting differentiation with 10 µM SB216763 instead of CHIR and using 1 µM A83-01 instead of SB on day 2 of differentiation. Figure 8f shows cells cultured by starting differentiation with 10 µM SB216763 instead of CHIR, replacing the medium with a medium supplemented with SB and DM on day 2 of differentiation, and using 50 µM dbcAMP instead of FSK from day 4 of differentiation.
Figure 8g shows cells cultured using 1 µM A83-01 instead of SB on day 2 of differentiation. Figure 8h shows cells cultured using 1 µM A83-01 and 0.1 µM LDN193189 instead of SB and DM.
[Figures 8i and 8j] Figure 8i shows cells supplemented with 50 µM dbcAMP instead of FSK from day 4 of differentiation. Figure 8j shows cells that were not supplemented with FSK from day 4 of differentiation.
[Figure 9] Figure 9 shows results of fluorescent immunostaining in the case of using 201B7 cells instead of 253G1 cells.
[Figure 10] Figure 10 shows results of FACS on cells stained with an anti-CD56 antibody before purification and after purification and results of observation under a fluorescence microscope. The ordinate depicts the fluorescence intensity of PE, and the abscissa depicts the fluorescence intensity of Alexa Fluoro 488.
[Figure 11] Figure 11 shows results of FACS on cells stained with an anti-CD56 antibody before purification and after purification and results of observation under a fluorescence microscope. The ordinate depicts the fluorescence intensity of PE, and the abscissa depicts the fluorescence intensity of Alexa Fluoro 488.

### Detailed Description

### 1. Term

### "Pluripotent stem cells"

The "pluripotent stem cells" according to the present invention refer to ES cells and cells potentially having pluripotent differentiation similar thereto, i.e., the ability to differentiate into various living tissues (all of the endoderm, the mesoderm and the ectoderm). The "pluripotent stem cells" are characterized by the expression of Oct3/4 and Nanog, which are transcriptional factors specifically expressed in pluripotent cells.

As for the ES cells, various mouse ES cell lines are available as mouse ES cells from, for example, inGenious Targeting Laboratory and Riken, Japan (AES001H-1 line), and various human ES cell lines are available as human ES cells from National Institutes of Health (NIH), Kyoto University and Cellartis SA.

Examples of the cells having pluripotent differentiation similar to that of the ES cells include "induced pluripotent stem cells" (in the present specification, also referred to as iPS cells). The iPS cells refer to cells obtained by reprogramming resulting from the introduction of particular factors (nuclear reprogramming factors) to mammalian somatic cells or undifferentiated stem cells. Currently, there are various "induced pluripotent stem cells". iPS cells established by the introduction of four factors, Oct3/4, Sox2, Klf4 and c-Myc, to mouse fibroblasts by Yamanaka et al. (Takahashi K, Yamanaka S., Cell, (2006) 126: 663-676) as well as human cell-derived iPS cells established by the introduction of similar four factors to human fibroblasts (Takahashi K, Yamanaka S., et al., Cell, (2007) 131: 861-872), Nanog-iPS cells established by the introduction of the four factors and subsequent screening with the expression of Nanog as an index (Okita, K., Ichisaka, T., and Yamanaka, S. (2007). Nature 448, 313-317), and iPS cells prepared by a c-Myc-free method (Nakagawa M, Yamanaka S., et al., Nature Biotechnology, (2008) 26, 101-106) can also be used. Furthermore, induced pluripotent stem cells established and prepared by the introduction of four factors, OCT3/4, SOX2, NANOG and LIN28, by Thomson et al. (Yu J., Thomson JA. et al., Science (2007) 318: 1917-1920), induced pluripotent stem cells prepared by Daley et al. (Park IH, Daley GQ. et al., Nature (2007) 451: 141-146), induced pluripotent stem cells prepared by Sakurada et al. (Japanese Patent Laid-Open No. 2008-307007), and the like can also be used.

In addition, any of induced pluripotent stem cells known in the art as described in all published papers (e.g., Shi Y., Ding S., et al., Cell Stem Cell, (2008) Vol. 3, Issue 5, 568-574; Kim JB., Scholer HR., et al., Nature, (2008) 454, 646-650; and Huangfu D., Melton, DA., et al., Nature Biotechnology, (2008) 26, No. 7, 795-797) or patents (e.g., Japanese Patent Laid-Open No. 2008-307007, Japanese Patent Laid-Open No. 2008-283972, US2008-2336610, US2009-047263, WO2007-069666, WO2008-118220, WO2008-124133, WO2008-151058, WO2009-006930, WO2009-006997 and WO2009-007852) can be used.

The pluripotent stem cells described in the present specification are preferably induced pluripotent stem cells, more preferably human induced pluripotent stem cells, particularly preferably a human induced pluripotent stem cell line 253G1 or 201B7.

### "Mesoderm"

The "mesoderm" is a cell population that develops to fill the space between the ectoderm and the endoderm and is characterized by the expression of pan-mesodermal markers BRACHYURY and TBX6. The mesoderm differentiates into the somitic mesoderm which makes up bone, cartilage, corium, skeletal muscle, etc., the intermediate mesoderm which makes up the kidney, adrenal gland, gonad, etc., and the lateral plate mesoderm which makes up the heart, vascular vessel, blood cells, etc. The somitic mesoderm that differentiates into skeletal muscle precursor cells or skeletal muscle cells is characterized by the expression of PAX3 and PAX7.

### "Skeletal muscle precursor cells"

The "skeletal muscle precursor cells" according to the present invention are undifferentiated cells induced from pluripotent stem cells and may differentiate into skeletal muscle cells, unless otherwise specified. The skeletal muscle precursor cells are characterized by the expression of a surface marker CD56 and/or a transcriptional factor PAX7 or the expression of CD29 and/or a transcriptional factor PAX7. Particularly, a skeletal muscle precursor cell preparation obtained by purification using CD56 (NCAM1) or CD29 (integrin subunit β1), etc. in the course of the method of the present invention has a high purity and concentration.

### "Myotube cells"

The "myotube cells" according to the present invention are cells induced from pluripotent stem cells through skeletal muscle precursor cells and are early cells in which the skeletal muscle precursor cells has started cell fusion, unless otherwise specified. The myotube cells refer to cells that have a plurality of nuclei and are at a stage where a myotome structure necessary for contractility has not yet been formed. The "myotube cells" are cells that may differentiate into skeletal muscle cells.

### "Skeletal muscle cells"

The "skeletal muscle cells" according to the present invention are cells induced from pluripotent stem cells through skeletal muscle precursor cells, unless otherwise specified, and are characterized by the expression of skeletal muscle cell markers such as MYOD, MYOG, MYH3, MYH7, MYH8, ACTA1, ACTN2, MYOD1 and MYF5. Particularly, the method of the present invention can produce a skeletal muscle cell preparation with a high purity and a high concentration.

### "GSK3β inhibitor"

The "GSK3β inhibitor" is a substance having inhibitory activity against GSK3β (glycogen synthase kinase 3β). GSK3 (glycogen synthase kinase 3), a serine/threonine protein kinase, participates in many signal pathways involved in the production of glycogen, apoptosis, the maintenance of stem cells, etc. GSK3 includes two isoforms, α and β. The "GSK3β inhibitor" used in the present invention is not particularly limited as long as the GSK3β inhibitor has GSK3β inhibitory activity. The GSK3β inhibitor may be a substance having GSK3α inhibitory activity in addition to the GSK3β inhibitory activity.

Examples of the GSK3β inhibitor include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), Kenpaullone, AR-AO144-18, TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), BIO (6-bromoindirubin-3-oxime), TWS-119 (3-[6-(3-aminophenyl)-7H-pyrrolo[2,3-d]pyrimidin-4-yloxy]phenol) and SB415286 (3-[(3-chloro-4-hydroxyphenyl)amino]-4-(2-nitrophenyl)-1H-pyrrole-2,5-dione). Also, an antisense oligonucleotide, siRNA, or the like against GSK3β mRNA can be used as the GSK3β inhibitor and is commercially available or can be synthesized according to a method known in the art.

The GSK3β inhibitor is preferably CHIR99021, SB216763, SB415286 or BIO, more preferably CHIR99021 (in the present specification, also referred to as CHIR) or a salt thereof.

### "ALK inhibitor"

The "ALK inhibitor" is a substance having inhibitory activity against a serine-threonine kinase ALK. Examples of the "ALK inhibitor" can include: ALK4, ALK5 and ALK7 inhibitors SB431542 (4-[4-(1,3-benzodioxol-5-yl)-5-(pyridin-2-yl)-1H-imidazol-2-yl]benzamide) and A83-01 (3-(6-methyl-2-pyridinyl)-N-phenyl-4-(4-quinolinyl)-1H-pyrazole-1-carbothioamide); an ALK2, ALK3 and ALK6 inhibitor LDN193189 (4-[6-(4-piperazin-1-yl-phenyl)-pyrazolo[1,5-α]pyrimidin-3-yl]-quinoline hydrochloride; and ALK5 inhibitors 2-(3-(6-methylpyridin-2-yl)-1H-pyrazol-4-yl)-1,5-naphthyridine; Wnt3a/BIO, BMP4, GW788388 (4-{4-[3-(pyridin-2-yl)-1H-pyrazol-4-yl]pyridin-2-yl}-N-(tetrahydro-2H-pyran-4-yl)benzamide), SM16, IN-1130 (3-((5-(6-methylpyridin-2-yl)-4-(quinoxalin-6-yl)-1H-imidazol-2-yl)methyl)benzamide), GW6604 (2-phenyl-4-(3-pyridin-2-yl-1H-pyrazol-4-yl)pyridine) and SB505124 (2-(5-benzo[1,3]dioxol-5-yl-2-tert-butyl-3H-imidazol-4-yl)-6-methylpyridine hydrochloride). The ALK inhibitor also includes an antibody and an antisense nucleic acid against ALK.

The ALK inhibitor is preferably SB431542 or A83-01, more preferably SB431542 (in the present specification, also referred to as SB) or a salt thereof.

### "cAMP inducer"

The "cAMP inducer" means a substance elevating the intracellular concentration of cAMP (cyclic adenosine monophosphate) or a substance activating cAMP signals. Examples of the cAMP inducer can include: (i) adenylate cyclase activators (e.g., forskolin ((3R)-(6AαH)dodecahydro-6β,10α,10bα-trihydroxy-3β,4aβ,7,7,10Aβ-pentamethyl-1-oxo-3-vinyl-1H-naphtho[2,1-b]pyran-5β-yl acetate), forskolin derivatives, and cholera toxin); (ii) cAMP derivatives (e.g., membrane-permeable analogs of cAMP (e.g., 8-bromo-cAMP (8-bromoadenosine-3',5'-cyclic monophosphate)); nonhydrolyzable analogs of cAMP (e.g., dbcAMP (dibutyryl cAMP)), Sp-cAMP (cAMP thiophosphate isomer), and 3-isobutyl-1-methylxanthine; and other cAMP derivatives; and (iii) PDE inhibitors (e.g., 3-isobutyl-1-methylxanthine (IBMX), pentoxifylline, rolipram, CP80633, CP102995, CP76593, Ro-20-1724 (4-[3-butoxy-4-methoxybenzyl]-2-imidazolidinone), theophylline, and denbufylline).

The cAMP inducer is preferably forskolin or a derivative thereof, or dbcAMP, more preferably forskolin (in the present specification, also referred to as FSK) or a salt thereof.

### "AMP kinase inhibitor"

The AMP kinase (AMP-activated protein kinase), a serine-threonine kinase, plays an important role as a sensor of intracellular energy. The "AMP kinase inhibitor" is an inhibitor of this AMP kinase. For example, dorsomorphin (6-[4-(2-piperidin-1-ylethoxy)phenyl]-3-pyridin-4-ylpyrazolo[1,5-a]pyrimidine), indirubin-3'-oxime, A-769662, AICAR (acadesine), phenformin HCl, GSK621, WZ4003 and HTH-01-015 are known.

The AMP kinase inhibitor is preferably dorsomorphin (in the present specification, also referred to as DM).

### "BMP signal inhibitor"

The BMP (bone morphogenetic protein) signal plays an important role in the course of development. The "BMP signal inhibitor" is an inhibitor of this BMP signal. For example, DM, DMH1, DMH2, K02288, LDN193189, LDN212854, LDN214117 and LDN193719 are known.

The BMP signal inhibitor is preferably LDN193719.

### "Growth factor"

The "growth factor" is an endogenous protein that promotes the differentiation and/or growth of particular cells. The growth factor used in the present invention is not particularly limited as long as the growth factor promotes differentiation and/or growth into skeletal muscle cells. Examples thereof can include fibroblast growth factor 2 (FGF2), fibroblast growth factor 1 (FGF1), hepatocyte growth factor (HGF), insulin-like growth factor 1 (IGF1), epithelial growth factor (EGF), interleukin 6 (IL6), and mixtures thereof.

The "growth factor" that is contained in or added to a medium is preferably FGF2, FGF1, HGF, EGF or IGF1, more preferably FGF2 or FGF1, further preferably FGF2.

In a particular step of the present invention, a medium "free from a growth factor" is used. In this context, the term "free from a growth factor" means that a growth factor in an amount that exerts an aggressive function for the induction of differentiation is neither contained nor added. Examples of the growth factor can include FGF2, FGF1, HGF, EGF and IGF1.

### "ROCK inhibitor"

The "ROCK inhibitor" means a substance inhibiting Rho kinase (ROCK: Rho-associated, coiled-coil containing protein kinase) and may be a substance inhibiting any of ROCK I and ROCK II. The ROCK inhibitor is not particularly limited as long as the ROCK inhibitor has the function described above. Examples of the ROCK inhibitor that can be used include: N-(4-pyridinyl)-4β-[(R)-1-aminoethyl]cyclohexane-1α-carboxamide (in the present specification, also referred to as Y-27632), fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-carboxamide (Wf-536), N-(1H-pyrrolo[2,3-b]pyridin-4-yl)-4PER(R)-1-aminoethyl]cyclohexane-carboamide (Y-30141), N-(3-{[2-(4-amino-1,2,5-oxadiazol-3-yl)-1-ethyl-1H-imidazo[4, 5-c]pyridin-6-yl]oxy}phenyl)-4-{[2-(4-morpholinyl)ethyl]-oxy}benzamide (GSK269962A) and N-(6-fluoro-1H-indazol-5-yl)-6-methyl-2-oxo-4-[4-(trifluoromethyl)phenyl]-3,4-dihydro-1H-pyridine-5-carboxamide (GSK429286A); antibodies (including functional fragments), antisense nucleic acids, and siRNA against ROCK; ROCK antagonists and dominant negative forms; and other ROCK inhibitors known in the art.

The ROCK inhibitor is preferably Y-27632.

### "Serum substitute"

In the present invention, it is preferred to use a "serum substitute" instead of serum. Examples of the "serum substitute" include Knockout Serum Replacement (KSR; Invitrogen Corp.), StemSure Serum Replacement (Wako Pure Chemical Industries, Ltd.), insulin-free B-27 supplement, N2-supplement, albumin (e.g., lipid-rich albumin), insulin, transferrin, fatty acid, collagen precursors, trace elements (e.g., zinc and selenium (e.g., sodium selenite)), 2-mercaptoethanol, 3'-thioglycerol and mixtures thereof (e.g., ITS-G).

The serum substitute is preferably insulin-free B-27 supplement, KSR, StemSure Serum Replacement or ITS-G. In the case of adding the serum substitute to a medium, its concentration in the medium is 0.01 to 10% by weight, preferably 0.1 to 2% by weight.

### "Medium"

Examples of the medium used in the present invention include BME medium, BGJb medium, CMRL 1066 medium, Glasgow MEM medium, Improved MEM Zinc Option medium, IMDM medium, Medium 199 medium, Eagle MEM medium, αMEM medium, DMEM medium (high glucose and low glucose), DMEM/F12 medium, Ham's medium, RPMI 1640 medium, Fischer's medium, and mixed media thereof.

The medium used in the present invention may be supplemented, if necessary, with, an amino acid, L-glutamine, GlutaMAX (product name), a nonessential amino acid, a vitamin, an antibiotic (e.g., antibiotic-antimycotic (in the present specification, also referred to as AA), penicillin, streptomycin, or a mixture thereof), an antimicrobial agent (e.g., amphotericin B), an antioxidant, pyruvic acid, a buffer, inorganic salts, and the like, in addition to the components described above. In the case of adding the antibiotic to the medium, its concentration in the medium is usually 0.01 to 20% by weight, preferably 0.1 to 10% by weight.

### Culture vessel and culture conditions

The cell culture according to the present invention is preferably performed by adhesion culture without the use of feeder cells. For the culture, a culture vessel is used such as a dish, a flask, a microplate, or a cell culture sheet such as OptiCell (product name) (Nalge Nunc International). The culture vessel is preferably surface-treated for improving adhesiveness to cells (hydrophilicity) or coated with a substrate for cell adhesion such as collagen, gelatin, poly-L-lysine, poly-D-lysine, laminin, fibronectin, Matrigel (e.g., BD Matrigel (Becton, Dickinson and Company Japan)) or vitronectin. The culture vessel is preferably a culture vessel coated with type I collagen, Matrigel, fibronectin, vitronectin or poly-D-lysine, etc., more preferably a culture vessel coated with Matrigel or poly-D-lysine.

The temperature of maintenance culture of pluripotent stem cells or culture for the induction of differentiation thereof is not particularly limited and is 30 to 40°C (e.g., 37°C). The concentration of carbon dioxide in the culture vessel is on the order of, for example, 5%.

### 2. Method for producing skeletal muscle precursor cells (induction of differentiation)

The present invention provides a method for producing skeletal muscle precursor cells from pluripotent cells. The said method comprises the steps of:
(1) culturing pluripotent stem cells in adhesion culture in a medium containing a GSK3β inhibitor for 1-4 days;
(2) culturing the cells obtained in the step (1) in adhesion culture in a medium containing an ALK inhibitor for 1-4 days; and
(3) culturing the cells obtained in the step (2) in adhesion culture in a medium containing a cAMP inducer for 9-26 days.

The pluripotent stem cells may be precultured for 1 day or overnight in a medium containing a ROCK inhibitor (e.g., Y-27632) or the like according to a routine method prior to differentiation culture. This can suppress cell death during differentiation culture.

### Step (1)

The pluripotent stem cells are first cultured in adhesion culture in a medium containing a GSK3β inhibitor for 1-4 days. The number of cells at the start of culture is not particularly limited and is 22000 to 150000 cells/cm², preferably 22000 to 50000 cells/cm², more preferably 22000 to 42000 cells/cm². The culture period is 1 day to 4 days, preferably 1 day to 3 days, particularly preferably 2 days.

The medium for use in this step is preferably DMEM/F12 medium, RPMI 1640 medium, or Improved MEM Zinc Option medium, more preferably RPMI 1640 medium.

The medium for use in this step is preferably supplemented with a serum substitute (e.g., insulin-free B-27 supplement or ITS-G) and an antibiotic (e.g., AA, penicillin, streptomycin, or a mixture thereof).

The culture vessel for use in the step (1) is preferably coated with Matrigel.

The concentration of the GSK3β inhibitor in the medium is appropriately set according to the type of the GSK3β inhibitor used. In the case of using CHIR as the GSK3β inhibitor, the concentration is usually 2 to 10 µM, preferably 2 to 8 µM, particularly preferably 5 to 7 µM. In the case of using BIO, the concentration is usually 0.2 to 0.8 µM, preferably 0.3 to 0.7 µM, particularly preferably 0.5 µM. In the case of using SB216763, the concentration is usually 6 to 14 µM, preferably 8 to 12 µM, particularly preferably 8 to 10 µM**.**

### Step (2)

The cells obtained in the step (1) are further cultured in adhesion culture in a medium containing an ALK inhibitor for 1-4 days. The culture period can be 1 day to 3 days, preferably 2 days.

The medium for use in this step is preferably RPMI 1640 medium supplemented with an antibiotic (e.g., AA, penicillin or streptomycin) and insulin-free B-27 supplement (Invitrogen Corp.) (in the present specification, RPMI 1640 medium supplemented with insulin-free B-27 supplement (Invitrogen Corp.) is also referred to as "B-27 medium").

The concentration of the ALK inhibitor in the medium is appropriately set according to the type of the ALK inhibitor used. In the case of using SB as the ALK inhibitor, the concentration is usually 0.3 to 30 µM, preferably 3 to 30 µM. In the case of using A83-01, the concentration is usually 1 to 5 µM, preferably 1 to 3 µM, particularly preferably 1 µM.

The medium in the step (2) may be further supplemented with an AMP kinase inhibitor (e.g., DM) to thereby induce skeletal muscle precursor cells with a higher purity and a higher concentration. The amount of the AMP kinase inhibitor added can be appropriately set. In the case of using DM as the AMP kinase inhibitor, the concentration is preferably 0.3 to 1 µM.

In this step, a BMP signal inhibitor may be used instead of or in addition to the AMP kinase inhibitor.

In the case of using LDN193189 as the BMP signal inhibitor, the concentration is usually 0.1 to 0.8 µM, preferably 0.1 to 0.5 µM, particularly preferably 0.1 µM.

### Step (3)

The cells obtained in the step (2) are further cultured in adhesion culture in a medium containing a cAMP inducer for 9-26 days and thereby differentiated into skeletal muscle precursor cells. The culture period is 9 days to 26 days, preferably 10 days to 25 days.

The concentration of the cAMP inducer in the medium is appropriately set according to the type of the cAMP inducer used, and usually used at a concentration of 0.1 to 1000 µM.

The cAMP inducer is preferably FSK, and its concentration in the medium is usually 1 to 50 µM, preferably 2 to 50 µM.

The media for use in the steps (1) to (3) may be appropriately supplemented with an additional component without impairing the object of the present invention. Preferably, no growth factor is added thereto. This is, particularly, because the addition of a growth factor that induces differentiation into skeletal muscle cells in the steps (1) to (3) promotes the induction of differentiation into skeletal muscle cells at once by skipping the stage of skeletal muscle precursor cells and might therefore have a negative effect on the purity or differentiation efficiency of cells.

The step (3) can be further divided into two steps. Hereinafter, these steps (3)-1 and (3)-2 will be described.

### Step (3)-1

In the step (3)-1, the cells obtained in the step (2) are cultured in a medium containing a cAMP inducer and thereby differentiated into the somitic mesoderm. The culture period is 2 to 5 days, preferably 3 to 4 days.

The medium for use in this step is preferably B-27 medium.

The concentration of the cAMP inducer in the medium is appropriately set according to the type of the cAMP inducer used, and usually used at a concentration of 0.1 to 1000 µM.

The cAMP inducer is preferably FSK, and its concentration in the medium is usually 1 to 50 µM, preferably 2 to 50 µM.

### Step (3)-2

In the step (3)-2, the cells obtained in the step (3)-1 are further cultured in a medium containing a cAMP inducer and thereby differentiated into skeletal muscle precursor cells. The culture period is preferably 7 days to 21 days. Approximately 3 weeks after the start of culture, skeletal muscle precursor cells positive for CD56 and/or Pax7 or positive for CD29 and/or Pax7 are induced.

The medium for use in this step is preferably a medium having a nutritional value equivalent to or higher than that of the medium used in the step (3)-1 and is preferably, for example, a medium having a glucose concentration equivalent to or higher than that of the medium used in the step (3)-1. In the case of using, for example, RPMI 1640 medium or B-27 medium in the step (3)-1, the medium for use in the step (3)-2 is preferably DMEM medium (high glucose). The glucose concentration in the medium for use in the step (3)-2 is preferably 2000 mg/L to 10000 mg/L, more preferably 3000 mg/L to 10000 mg/L, particularly preferably 4000 mg/L to 10000 mg/L. The medium for use in this step is preferably supplemented with an antibiotic (e.g., AA, penicillin, streptomycin, or a mixture thereof).

The concentration of the cAMP inducer in the medium is appropriately set according to the type of the cAMP inducer used, and usually used at a concentration of 0.1 to 1000 µM.

The cAMP inducer is preferably FSK, and its concentration in the medium is usually 1 to 50 µM, preferably 2 to 50 µM.

Whether or not the cells obtained in the step (3)-2 are positive for CD56 and/or Pax7 or positive for CD29 and/or Pax7 can be confirmed by a method known per se in the art, such as immunostaining or real-time PCR (in the present specification, also referred to as RT-PCR).

### 3. Purification of skeletal muscle precursor cells

The obtained skeletal muscle precursor cells can be purified through the use of a surface marker CD56 (NCAM1) or CD29 (integrin subunit β1). The purification can be performed by use of a method known per se in the art, for example, anti-CD56 antibody-immobilized beads. The anti-CD56 antibody-immobilized beads may be obtained as a commercially available product (e.g., CD56 MicroBeads (Miltenyi Biotec)). Alternatively, the antibody-immobilized beads may be prepared using EasySep (trade name) Human PE Positive Selection Kit (StemCell Technologies Inc.).

The skeletal muscle precursor cells may be purified on the basis of CDH15 (M-cadherin), CD82, CD34, MET, CXCR4, or ITGA7 (integrin subunit α7), in addition to CD56 and CD29 and may be further purified using Caveolin-1, CTR, ErbB receptor, necdin, Megf10, p75NTR/BDNFc, sphingomyelin, syndecan 3/4, VCAM-1, or nestin.

### 4. Dispersion and cryopreservation of skeletal muscle precursor cells

According to the method described herein, a highly pure skeletal muscle precursor cell preparation (culture product) can be obtained. The cells or the preparation obtained in the step (3) can be frozen to prepare a frozen cell preparation comprising skeletal muscle precursor cells.

The obtained cells or preparation can be easily dispersed (dispersed in a state of single cells) by enzymatic treatment. A commercially available reagent for cell dispersion or cell-dispersing solution (e.g., ACCUMAX or Accutase (both from Innovative Cell Technologies, Inc.)) can be used in the dispersion. The dispersion can be performed by a method known per se in the art, for example, by adding a commercially available reagent for cell dispersion or cell-dispersing solution to a culture vessel, reacting for a given time, then adding a culture solution or the like thereto, and pipetting the culture solution several times.

In the present specification, the "dispersing solution" means these reagents for cell dispersion or cell-dispersing solutions, etc. A preparation (culture product) comprising skeletal muscle precursor cells in a dispersing solution can also be cryopreserved.

An enzyme for cells and/or for protein cleavage can be used as the enzyme for use in the "enzymatic treatment" described in the present specification. For example, a commercially available enzyme can be used such as Celase, Detach Kit, Accutase, Accutase LZ, Accumax, Neonatal Cardiomyocyte Isolation System, STEMxyme, Protease, Neutral, Elastase, Papain Dissociation System, Neonatal Cardiomyocyte Isolation System, Trypsin/EDTA Solution, FACSmax Cell Dissociation Solution, Detachin, Collagenase, Collagenase type I, II, III and IV, Cell Isolation Optimizing System, Dispase I and II.

A cell preparation (culture product) obtainable by the method described in the present specification is also described. The purity of the cells of interest in the cell preparation (culture product) can be evaluated by a method known per se in the art. For example, the proportion of the cells of interest in the cell preparation (culture product) can be evaluated by analysis such as flow cytometry or cellular imaging. The proportion of cells expressing the gene of interest can be evaluated by gene expression profile analysis on each one of the cells in the cell preparation (culture product).

The flow cytometry is a technique of optically analyzing individual cells, which involves injecting a dispersion fluid containing cells labeled with an appropriate label into a capillary where the dispersion fluid then runs as a narrow stream, and measuring detection intensity based on the label to count the cells of interest. A measurement apparatus therefor is called flow cytometer. For example, by use of FACSAria from Becton, Dickinson and Company (BD), cells stained with a fluorescent antibody are allowed to run through a liquid stream and pass through the focus of laser light, and the amount of an antigen present on cell surface can be quantitatively measured by the measurement of fluorescence emitted by each individual cell. This technique can detect scattered light or fluorescent dyes associated with the forms and functions of a plurality of cells at the same time. Therefore, if a sample contains a plurality of cell populations, the proportion of cells expressing a particular marker protein or gene, included in heterogeneous cell populations can be calculated by using a plurality of parameters.

The cellular imaging is an approach which involves obtaining cell images from culture plates or glass slides using an apparatus, for example, In Cell Analyzer 600 from GE Healthcare Japan Corp., and computer-analyzing the images. This technique can obtain objective and a great deal of information on each cell in a cell population and can calculate the proportion of cells having particular properties.

Single-cell analysis can be combined with various analysis apparatuses to attain various analyses. The qPCR analysis of a selected gene can be conducted by separating a heterogeneous cell population into single cells in C1 from Fluidigm Corp. and using a real-time PCR system such as Fluidigm Corp. BioMark or ABI7900 as an analysis apparatus. Alternatively, gene expression levels can be comprehensively measured by using a next-generation sequencer Ion Proton from Thermo Fisher Scientific Inc. This technique can obtain a gene expression profile for each of the single cells and can therefore calculate the proportion of cells expressing a particular gene in the heterogeneous cell population.

The cell preparation (culture product) obtained by the method described in the present specification comprises at least approximately 99%, at least approximately 98%, at least approximately 97%, at least approximately 96%, at least approximately 95%, at least approximately 94%, at least approximately 93%, at least approximately 92%, at least approximately 91%, at least approximately 90%, at least approximately 89%, at least approximately 88%, at least approximately 87%, at least approximately 86%, at least approximately 85%, at least approximately 84%, at least approximately 83%, at least approximately 82%, at least approximately 81%, at least approximately 80%, at least approximately 79%, at least approximately 78%, at least approximately 77%, at least approximately 76%, at least approximately 75%, at least approximately 74%, at least approximately 73%, at least approximately 72%, at least approximately 71%, at least approximately 70%, at least approximately 69%, at least approximately 68%, at least approximately 67%, at least approximately 66%, at least approximately 65%, at least approximately 64%, at least approximately 63%, at least approximately 62%, at least approximately 61%, at least approximately 60%, at least approximately 59%, at least approximately 58%, at least approximately 57%, at least approximately 56%, at least approximately 55%, at least approximately 54%, at least approximately 53%, at least approximately 52%, at least approximately 51%, at least approximately 50%, at least approximately 45%, at least approximately 40%, at least approximately 35%, at least approximately 30%, at least approximately 29%, at least approximately 28%, at least approximately 27%, at least approximately 26%, at least approximately 25%, at least approximately 24%, at least approximately 23%, at least approximately 22%, at least approximately 21%, at least approximately 20%, at least approximately 19%, at least approximately 18%, at least approximately 17%, at least approximately 16%, at least approximately 15%, at least approximately 14%, at least approximately 13%, at least approximately 12%, at least approximately 11%, at least approximately 10%, at least approximately 9%, at least approximately 8%, at least approximately 7%, at least approximately 6%, at least approximately 5%, at least approximately 4%, at least approximately 3%, at least approximately 2%, or at least approximately 1%, preferably 40% or more, more preferably 50% or more, further preferably 60% or more, of the cells of interest (e.g., skeletal muscle precursor cells or skeletal muscle cells). The purity of the cell preparation (culture product) can be calculated with no consideration given to feeder cells remaining in the cell preparation (culture product).

The frozen cell preparation described herein is a frozen cell preparation comprising skeletal muscle precursor cells obtained by the method of the present invention.

The frozen cell preparation is a frozen cell preparation comprising skeletal muscle precursor cells obtained by the method of the present invention in a dispersing solution.

A frozen cell preparation described herein is a frozen cell preparation comprising CD56- and/or PAX7-positive skeletal muscle precursor cells induced from pluripotent stem cells in a dispersing solution, the frozen cell preparation comprising the skeletal muscle precursor cells in a cell proportion of 30% or more, preferably 40% or more, more preferably 50% or more as measured by flow cytometry.

Alternatively, a frozen cell preparation described herein is a frozen cell preparation comprising CD29- and/or PAX7-positive skeletal muscle precursor cells induced from pluripotent stem cells in a dispersing solution, the frozen cell preparation comprising the skeletal muscle precursor cells in a cell proportion of 30% or more, preferably 40% or more, more preferably 50% or more as measured by flow cytometry.

The frozen cell preparation can be thawed, if necessary, and used in the production of skeletal muscle cells. Specifically, cells obtained by thawing the frozen preparation can be subjected to method (2-1) mentioned later, etc. to thereby induce skeletal muscle cells. Such a frozen cell preparation comprising skeletal muscle precursor cells is also described.

The frozen cell preparation comprising skeletal muscle precursor cells is preferably prepared by freezing an enzymatically treated dispersion of the skeletal muscle precursor cell preparation (culture product) obtained by the method of the present invention, preferably a dispersion thereof in the state of single cells. The preparation can be performed by a method known per se in the art, for example, by freezing a solution of the dispersed skeletal muscle precursor cells resuspended using Cellbanker 2 (Nippon Zenyaku Kogyo Co., Ltd.).

### 5. Method for producing skeletal muscle cells (induction of differentiation)

The present invention also provides a method for producing skeletal muscle cells, comprising inducing differentiation of skeletal muscle precursor cells obtained in the step (3) or skeletal muscle precursor cells obtained by thawing a frozen cell preparation obtained from cells obtained in step (3), into skeletal muscle cells. The method for inducing differentiation of the skeletal muscle precursor cells into skeletal muscle cells includes the following two methods.

### Method for inducing differentiation of skeletal muscle precursor cells into skeletal muscle cells - 1 (method 1)

In method 1, the skeletal muscle precursor cells obtained in the step (3) or the skeletal muscle precursor cells obtained by thawing the frozen cell preparation are cultured in adhesion culture in a medium containing a cAMP inducer and thereby differentiated into skeletal muscle cells. The culture period can be set to 4 weeks or longer and is usually 4 weeks to 6 months, preferably 4 weeks to 2 months, more preferably 4 weeks to 6 weeks.

It is preferred to disperse the skeletal muscle precursor cells in advance, followed by plating over the medium. This can produce highly pure skeletal muscle cells.

Preferred examples of the medium that can be used in this method are the same as those listed in the step (3)-2. The medium for use in this method is preferably DMEM medium (high glucose). The medium for use in this process is preferably supplemented with a cAMP inducer (e.g., FSK or dbcAMP), a serum substitute (e.g., KSR or ITS-G) and an antibiotic (e.g., AA, penicillin or streptomycin).

The concentration of the cAMP inducer in the medium is appropriately set according to the type of the cAMP inducer used, and usually used at a concentration of 0.1 to 1000 µM.

The cAMP inducer is preferably FSK or dbcAMP, and its concentration in the medium is usually 1 to 50 µM, preferably 2 to 50 µM.

The culture vessel in this method is preferably coated with Matrigel, fibronectin or poly-D-lysine.

### Method for inducing differentiation of skeletal muscle precursor cells into skeletal muscle cells - 2 (method 2)

Method 2 comprises a method for producing myotube cells from the skeletal muscle precursor cells (method 2-1) and a method for producing skeletal muscle cells from the myotube cells (method 2-2).

### Method for producing myotube cells from skeletal muscle precursor cells (method 2-1)

Method 2-1 is a method for producing myotube cells, comprising culturing the skeletal muscle precursor cells obtained in the step (3) or the skeletal muscle precursor cells obtained by thawing a frozen cell preparation obtained from cells obtained in step (3), in adhesion culture in a medium containing a growth factor for 7-20 days to induce differentiation into myotube cells. The culture period is 7 days to 20 days, preferably 10 to 14 days. The culture is preferably performed with the medium replaced every 2 to 3 days.

It is preferred to disperse the skeletal muscle precursor cells in advance, followed by plating over the medium. This can produce highly pure skeletal muscle cells.

The growth factor for use in this method is preferably FGF2, FGF1, HGF, EGF or IGF1, more preferably FGF1 or FGF2, further preferably FGF2.

Preferred examples of the medium that can be used in this method are the same as those listed in the step (3)-2. The medium for use in this method is preferably DMEM medium (high glucose or low glucose), DMEM/F12 medium, RPMI 1640 medium or Improved MEM Zinc Option medium, more preferably DMEM (high glucose) medium. The medium for use in this step is preferably supplemented with a cAMP inducer (e.g., FSK), a serum substitute (e.g., KSR or ITS-G) and an antibiotic (e.g., AA, penicillin or streptomycin).

The concentration of the growth factor in the medium is appropriately set according to the type of the growth factor used and is usually approximately 0.1 nM to 1000 µM, preferably approximately 0.1 nM to 100 µM. The concentration of EGF is approximately 5 to 2000 ng/ml (i.e., approximately 0.8 to 320 nM), preferably approximately 5 to 1000 ng/ml (i.e., approximately 0.8 to 160 nM), more preferably approximately 10 to 1000 ng/ml (i.e., approximately 1.6 to 160 nM). The concentration of FGF1 or FGF2 is approximately 5 to 2000 ng/ml (i.e., approximately 0.3 to 116 nM), preferably approximately 10 to 1000 ng/ml (i.e., approximately 0.6 to 58 nM), more preferably approximately 10 to 1000 ng/ml (i.e., approximately 0.6 to 58 nM).

The concentration of the growth factor in the medium is particularly preferably set to 50 ng/mL in the case of using, for example, FGF1, 10 ng/mL in the case of using FGF2, 50 ng/mL in the case of using EGF, 50 ng/mL in the case of using IGF1, and 50 ng/mL in the case of using HGF.

The medium preferably further contains a cAMP inducer. The addition of the cAMP inducer promotes the induction of differentiation into skeletal muscle cells. The concentration of the cAMP inducer in the medium is appropriately set according to the type of the cAMP inducer used, and usually used at a concentration of 0.1 to 1000 µM.

The cAMP inducer is preferably FSK or dbcAMP, and its concentration in the medium is usually 1 to 50 µM, preferably 2 to 50 µM, for FSK, and usually 10 to 100 µM, preferably 10 to 50 µM, for dbcAMP.

The culture vessel for use in this method is preferably coated with Matrigel, laminin or fibronectin.

In the case of obtaining the skeletal muscle precursor cells by thawing the frozen cell preparation in the method 1 and the method 2-1, it is preferred to add a ROCK inhibitor (e.g., Y-27632) to an initial medium after the thawing. The concentration of the ROCK inhibitor in the medium is appropriately set according to the type of the ROCK inhibitor used. In the case of using Y-27632 as the ROCK inhibitor, the concentration is usually 0.1 µM to 1000 µM, preferably 1 µM to 100 µM, more preferably 0.3 µM to 30 µM.

### Method for producing skeletal muscle cells from myotube cells (method 2-2)

Method 2-2 is a method for producing skeletal muscle cells, comprising culturing the myotube cells obtained by the method 2-1 in a medium free from a growth factor to induce differentiation into skeletal muscle cells. The medium can be basically the same as the medium used in the method 2-1 except that the medium for use in this method is free from a growth factor. The term "free from a growth factor" means that, as mentioned above, a growth factor in an amount that exerts an aggressive function for the induction of differentiation is neither contained nor added. The culture period is not particularly limited. The culture period can be set to 2 weeks or longer and is usually 2 weeks to 6 months, preferably 2 weeks to 6 weeks. The culture is preferably performed with the medium replaced every 2 to 3 days.

The method described in the method 2 can also produce skeletal muscle cells from skeletal muscle precursor cells that are not limited to the skeletal muscle precursor cells obtained in the step (3) and the skeletal muscle precursor cells obtained by thawing the frozen cell preparation. Specifically, a method for producing skeletal muscle cells is described, comprising the steps of: (I) culturing skeletal muscle precursor cells in a medium containing a growth factor; and (II) culturing the cells obtained in the step (I) in a medium free from a growth factor.

More specifically, the method for producing skeletal muscle cells from pluripotent stem cells includes the following methods.

### Method for producing skeletal muscle cells from pluripotent stem cells - A (method A)

This method comprises the steps of:
(A1) culturing pluripotent stem cells in a medium containing a GSK3β inhibitor (particularly, CHIR, SB216763 or BIO);
(A2) culturing the cells obtained in the step (A1) in a medium containing an ALK inhibitor (particularly, SB431542 or A83-01);
(A3) culturing the cells obtained in the step (A2) in a medium containing a cAMP inducer (particularly, forskolin or dbcAMP); and
(A4) culturing the skeletal muscle precursor cells obtained in the step (A3) in a medium containing a cAMP inducer (particularly, forskolin) to induce differentiation into skeletal muscle cells.

The number of days from the start of differentiation is step (A1): the 0th to 2nd days from the starting day of differentiation/step (A2): the 2nd to 4th days therefrom/step (A3): the 4th to 21st days therefrom/step (A4): after the step (A3), as an example. In this context, the step (A4) corresponds to method 1.

### Method for producing skeletal muscle cells from pluripotent stem cells - B (method B)

This method comprises the steps of:
(B1) culturing pluripotent stem cells in a medium containing a GSK3β inhibitor (particularly, CHIR, SB216763 or BIO);
(B2) culturing the cells obtained in the step (B1) in a medium containing an ALK inhibitor (particularly, SB431542 or A83-01);
(B3) culturing the cells obtained in the step (B2) in a medium containing a cAMP inducer (particularly, forskolin or dbcAMP);
(B4) culturing the skeletal muscle precursor cells obtained in the step (B3) in a medium containing a growth factor (particularly, FGF1, FGF2, EGF, IGF1 or HGF (preferably FGF1 or FGF2, more preferably FGF2)) to induce differentiation into myotube cells; and
(B5) culturing the myotube cells obtained in the step (B4) in a medium free from a growth factor to induce differentiation into skeletal muscle cells.

The number of days from the start of differentiation is step (B1): the 0th to 2nd days from the starting day of differentiation/step (B2): the 2nd to 4th days therefrom/step (B3): the 4th to 21st days therefrom/step (B4): the 21st to 35th days therefrom/step (B5): after the step (B4), as an example. In this context, the steps (B4) and (B5) correspond to method 2-1 and method 2-2, respectively.

The steps (A3) and (B3) each correspond to the step (3) and can each be divided into two steps corresponding to the steps (3)-1 and (3)-2. In the two divided steps, the number of days of each step is step corresponding to the step (3)-1: the 4th to 7th days from the starting day of differentiation/step of corresponding to the step (3)-2: the 7th to 21st days therefrom, as an example.

In both the methods A and B, the skeletal muscle precursor cells obtained in the steps (A3) and (B3), respectively, can be frozen before the steps (A4) and (B4), respectively, to prepare a frozen cell preparation comprising skeletal muscle precursor cells. Also, the cells or the preparation obtained in the step (A3) or (B3) can be easily dispersed (dispersed in the state of single cells) by enzymatic treatment. The dispersion method is as described above. The skeletal muscle precursor cells or the preparation for use in the step (A4) or (B4) is preferably prepared by freezing an enzymatically treated dispersion of the skeletal muscle precursor cell preparation (culture product) obtained by the method of the present invention, preferably a dispersion thereof in the state of single cells. Even in the case of dispersing the skeletal muscle precursor cells obtained in the step (A3) or (B3) or further freezing the dispersion to prepare a frozen cell preparation, skeletal muscle cells can be produced according to the same culture method and culture period as in the steps (A4), (B4) and (B5) except for the operation of thawing the frozen cell preparation.

The skeletal muscle precursor cells and the skeletal muscle cells obtained by the methods of the present invention have a high purity and are useful as a cell preparation for regenerative medicine. These cells are also useful for screening for various drugs. For use as the cell preparation for regenerative medicine, examples of the administration method include systemic administration and local administration to appropriate tissues such as muscle tissues or subcutaneous tissues. Examples of the dosage form include solutions typified by injections, liniments, and forms thereof encapsulated in drug release devices. Examples of the screening for various drugs include screening for useful reagents for the induction of differentiation into skeletal muscle cells. Also, disease model cells can be prepared from genetically engineered pluripotent stem cells and thereby used in screening for therapeutic drugs for various diseases or the evaluation of their activity, drug efficacy and toxicity, etc. The therapeutic drugs for various diseases that can be to be screened for, for example, include various drugs including not only low-molecular organic compounds but peptides, proteins, antibodies (including whole antibodies, partial antibodies and combinations thereof), nucleic acids, improved antibodies, cells, and genetically engineerable nucleic acids and proteins, and combinations thereof. Examples of the disease that may be targeted may include: myogenic diseases such as various muscular dystrophies, various myopathies, mitochondrial disease, and glycogen storage disease; and sarcopenia, cachexia, and muscle atrophy caused by reduced muscle activity associated with a bedridden state or fracture. Further examples of the target disease may include neurogenic diseases such as infantile spinal muscular atrophy, Charcot-Marie-Tooth disease, congenital hypomyelinating leukodystrophy, and amyotrophic lateral sclerosis (ALS).

### Examples

Hereinafter, the present invention will be specifically described with reference to Reference Example and Examples. However, the present invention is not limited by these examples.

### Reference Example 1: Maintenance culture of 253G1 cells as human iPS cells

The human iPS cells used were 253G1 cells (Nature Biotechnology 26, 101-106).

For the maintenance culture of the 253G1 cell, the medium used was Essential 8 medium (Invitrogen Corp.; in the present specification, also referred to as E8 medium) supplemented with 1% AA or 0.5% penicillin-streptomycin solution, and the culture vessel used was coated with vitronectin (VTN-N, Invitrogen Corp.).

In this Reference Example and Examples given below, cell culture was performed at 37°C under 5% CO₂.

In order to prepare cells for the induction of differentiation described in Examples given below, the maintenance-cultured 253G1 cells were dispersed in 0.5 mM ethylenediamine tetraacetate in phosphate-buffered saline and then plated over E8 medium supplemented with 10 µM Y-27632 (Wako Pure Chemical Industries, Ltd.) in a 96-well, 48-well, 24-well, 12-well or 6-well plate coated with Matrigel. On the day following the plating, the medium was replaced with E8 medium.

### Example 1: Induction of differentiation of human iPS cells into somitic mesoderm

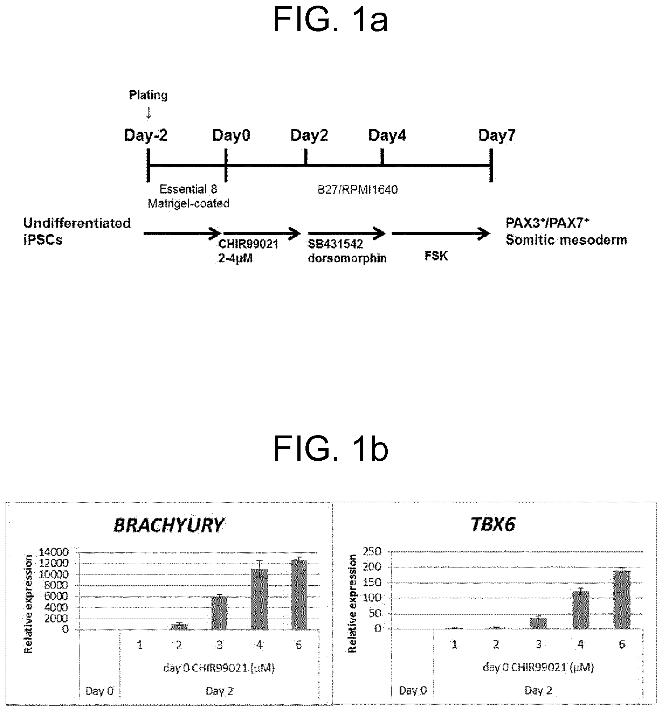

Figure 1a summarizes a method for inducing differentiation of undifferentiated iPS cells into the somitic mesoderm as described in Example 1. Use of this method for inducing differentiation induces the somitic mesoderm from undifferentiated iPS cells by way of the mesoderm.

### <1-1. Induction of differentiation of human iPS cells into mesoderm>

According to Reference Example 1, 253G1 cells were plated over a Matrigel-coated culture vessel. Two days thereafter, the E8 medium was replaced with B-27 medium supplemented with 1 to 6 µM CHIR and 1% AA to start differentiation (the starting day of differentiation was defined as day 0 of differentiation).

On day 2 of differentiation, change in the expression of pan-mesodermal marker genes BRACHYURY and TBX6 was examined. The change in gene expression was analyzed by the following procedures: total RNA was prepared using RNeasy 96 Kit or RNeasy Mini Kit (Qiagen N.V.), and cDNA was then synthesized from the total RNA using reverse transcriptase (PrimeScript RT reagent kit, Takara Bio Inc.). The obtained cDNA was subjected to real-time PCR using a Taqman probe (Applied Biosystems, Inc.). A sample prepared from the 253G1 cells before the start of differentiation was used as a reference sample. The gene expression levels of BRACHYURY and TBX6 were calculated by the comparative Ct method.

As a result, as shown in Figure 1b, BRACHYURY and TBX6 expression was found to increase in a CHIR concentration-dependent manner within the range of 1 to 6 µM on day 2 of differentiation. In Figure 1b, the gene expression levels of BRACHYURY and TBX6 on day 2 of differentiation are indicated by ratios to their expression levels defined as 1 in undifferentiated iPS cells.

### <1-2. Induction of differentiation of mesoderm into somitic mesoderm>

On day 2 of differentiation, the medium was replaced with B-27 medium supplemented with 1% AA and 10 µM SB (and 0.3 to 1 µM DM). On day 4 of differentiation, the medium was further replaced with B-27 medium supplemented with 1% AA and 10 µM FSK. The culture was continued until day 7 of differentiation.

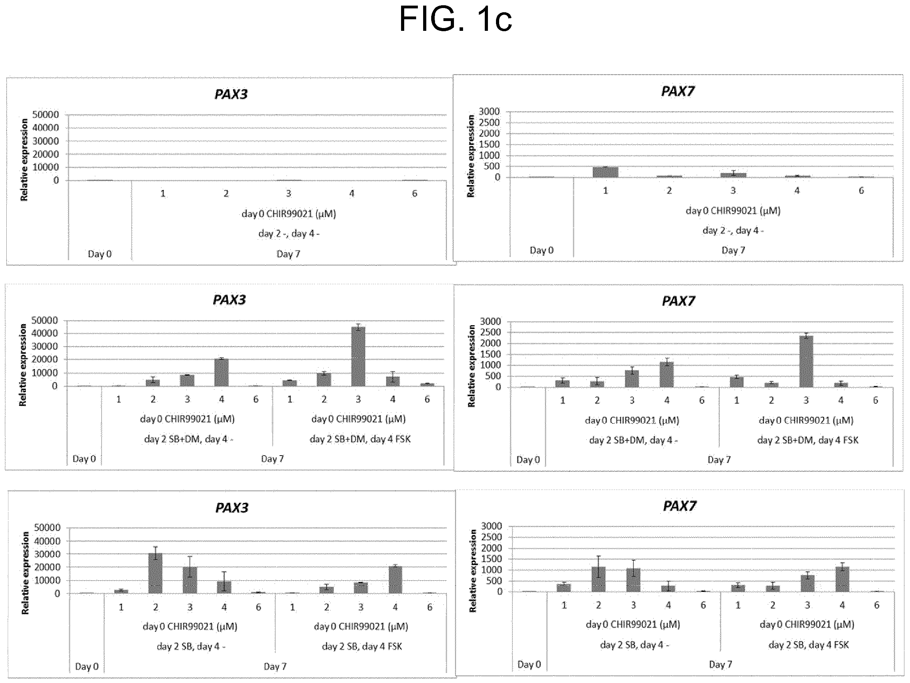

On day 7 of differentiation, the expression levels of somitic mesoderm marker genes PAX3 and PAX7 were measured. As a result, as shown in Figure 1c, the somitic mesoderm marker genes were found to exhibit high expression when the cells were cultured in the medium containing 2 to 4 µM CHIR from days 0 to 2 of differentiation, then cultured in the medium containing SB (and DM) from days 2 to 4 of differentiation, and further cultured in the medium containing FSK from days 4 to 7 of differentiation. In Figure 1c, the gene expression levels of PAX3 and PAX7 on day 7 of differentiation are indicated by ratios to their expression levels defined as 1 in 253G1 cells before the start of differentiation.

### Example 2: Differentiation of somitic mesoderm into skeletal muscle

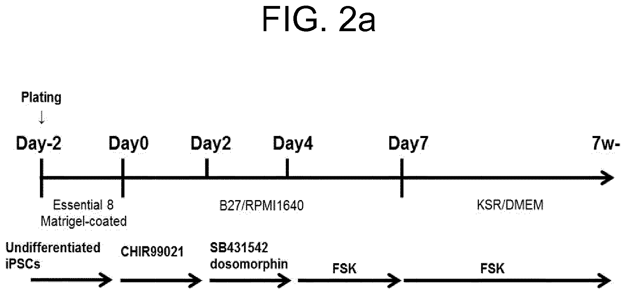

Figure 2a summarizes a method for inducing differentiation of undifferentiated iPS cells into skeletal muscle cells by way of the mesoderm and the somitic mesoderm. Use of this induction method induces differentiation of undifferentiated iPS cells into skeletal muscle cells.

### <Induction of differentiation of somitic mesoderm into skeletal muscle cells>

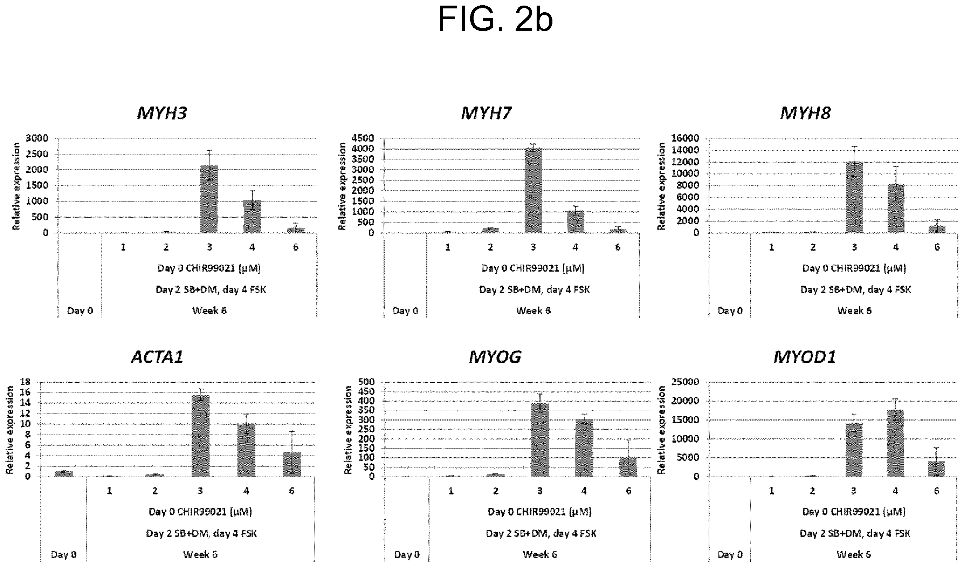

On day 7 of differentiation, the medium of the cells differentiated into the somitic mesoderm was replaced with DMEM (high glucose) medium supplemented with 10 µM FSK, 5% KSR, 1% AA and 1% ITS-G. The culture was continued with the medium replaced every 2 to 3 days so that differentiation into skeletal muscle cells was induced. Spontaneously contracting cells were induced from week 5 of differentiation. On week 6 of differentiation, the expression levels of skeletal muscle cell markers were measured by real-time PCR. As a result, as shown in Figure 2b, the gene expression levels of the skeletal muscle cell markers (specifically, embryonic skeletal muscle myosin heavy chains (MYH3 and MYH8), β myosin heavy chain (MYH7), skeletal muscle actin (ACTA1), and skeletal muscle transcriptional factors (MYOG and MYOD1)) were found to increase on week 6 of differentiation. In Figure 2b, the gene expression levels of the skeletal muscle cell markers are indicated by ratios to their expression levels defined as 1 in 253G1 cells before the start of differentiation.

### Example 3: Differentiation of skeletal muscle precursor cells induced from iPS cell into skeletal muscle and preparation of frozen stock

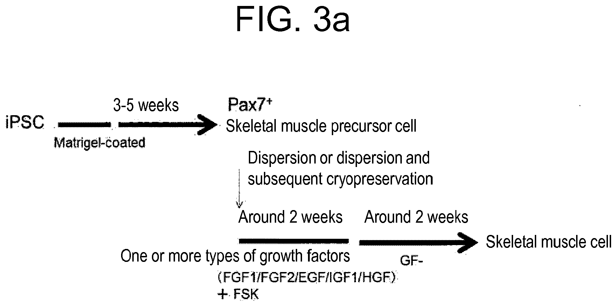

Figure 3a summarizes a method for inducing differentiation of skeletal muscle precursor cells induced from undifferentiated iPS cells into skeletal muscle cells by way of dispersion and plating or dispersion and subsequent cryopreservation. Use of this method for inducing differentiation enables the induction of differentiation of skeletal muscle precursor cells differentiated by induction from undifferentiated iPS cells, into skeletal muscle in a culture vessel different from the culture vessel used for inducing the skeletal muscle precursor cells, and the induction of skeletal muscle cells from cryopreserved skeletal muscle precursor cells.

### <3-1. Expression of skeletal muscle precursor cell and skeletal muscle cell markers in cells on week 4 of differentiation>

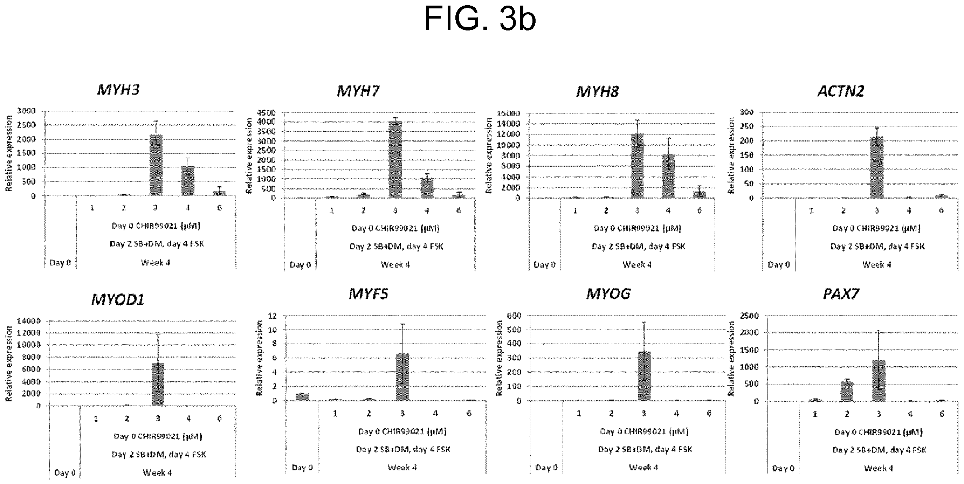

The expression levels of a skeletal muscle precursor cell marker (PAX7) and skeletal muscle markers (MYH3, MYH7, MYH8, ACTN2, MYOD1, MYF5 and MYOG) in the cells cultured in the same way as the method described in Example 2 were measured on week 4 of differentiation. As shown in Figure 3b, the cells on week 4 of differentiation were found to express the skeletal muscle markers in addition to the skeletal muscle precursor cell marker. This demonstrated that the cells differentiated into cells of skeletal muscle lineage on week 4 of differentiation. In Figure 3b, the gene expression levels of the skeletal muscle precursor cell marker and the skeletal muscle cell markers are indicated by ratios to their expression levels defined as 1 in 253G1 cells before the start of differentiation.

### <3-2. Dispersion and plating of skeletal muscle precursor cells>

Study was made on whether the cells could be dispersed and plated over a fresh culture vessel on weeks 3 to 5 of differentiation after day 7 of differentiation. Specifically, the cells cultured in the same way as the method described in Example 2 were dispersed and plated on week 3, 4 or 5 of differentiation. Study was made on whether or not skeletal muscle cells could be induced from the cells thus plated. As a result, differentiation into skeletal muscle cells was able to be induced after dispersion and plating even when the cells were dispersed and plated on week 3, 4 or 5 of differentiation. This demonstrated that skeletal muscle precursor cells that may differentiate into skeletal muscle cells can be obtained even when cells on weeks 3 to 5 of differentiation are dispersed and plated over a fresh culture vessel. As the culture period gets longer, cells are less susceptible to enzymatic digestion and more difficult to disperse due to components secreted by the cells. Therefore, in the case of using an enzyme solution as a dispersing solution, weeks 3 to 4 of differentiation were suitable for dispersion and plating.

### <3-3. Cryopreservation of skeletal muscle precursor cells>

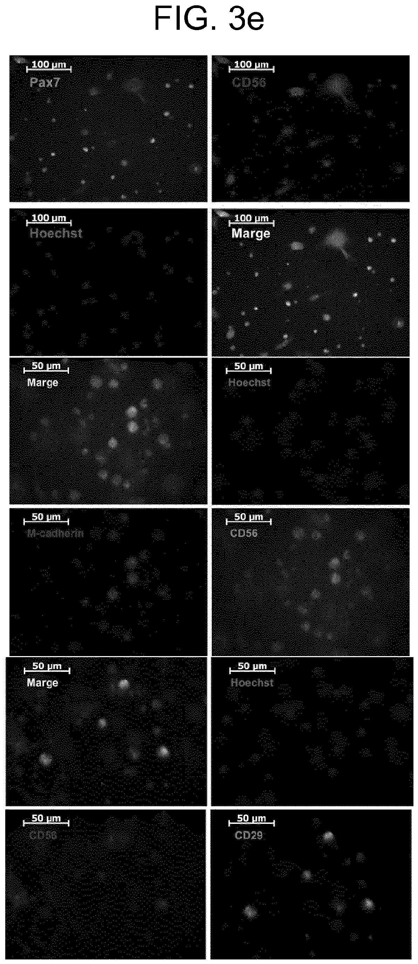

In order to study whether the cells cultured in the same way as the method described in Example 2 could be cryopreserved on weeks 3 to 4 of differentiation, the cells on week 3 or 4 of differentiation were dispersed in ACCUMAX and frozen using Cellbanker 2 (Nippon Zenyaku Kogyo Co., Ltd.) to prepare a frozen cell preparation. In order to confirm whether skeletal muscle precursor cells could be obtained from the frozen cell preparation, the frozen cell preparation was quickly thawed on a water bath of 37°C, then plated over DMEM (high glucose) medium supplemented with 10 µM Y-27632, 5% KSR, 1% AA and 1% ITS-G in a poly-D-lysine-coated plate (Corning Inc.), and cultured for 2 days. After the culture, the expression of skeletal muscle precursor cell markers PAX7 and CD56 was confirmed by the fluorescent immunostaining method using an anti-PAX7 antibody (antibody manufactured by Abcam plc.) and an anti-CD56 antibody (antibody manufactured by Santa Cruz Biotechnology, Inc.). As a result, as shown in Figure 3e, small cells were positive for PAX7 and/or CD56. Also, the expression of CD56 (using an antibody manufactured by Santa Cruz Biotechnology, Inc.) and another known skeletal muscle precursor cell marker M-cadherin (using an antibody manufactured by Santa Cruz Biotechnology, Inc.), or CD56 (using an antibody manufactured by Santa Cruz Biotechnology, Inc.) and CD29 (using an antibody manufactured by Abcam plc.) was similarly examined. As a result, M-cadherin or CD29 was found to be also expressed in small cells, at least some of which expressed CD56 together therewith. This demonstrated that skeletal muscle precursor cells can be obtained from the frozen cell preparation.

### <3-4. Differentiation of skeletal muscle precursor cells obtained from frozen cell preparation into myotube cells>

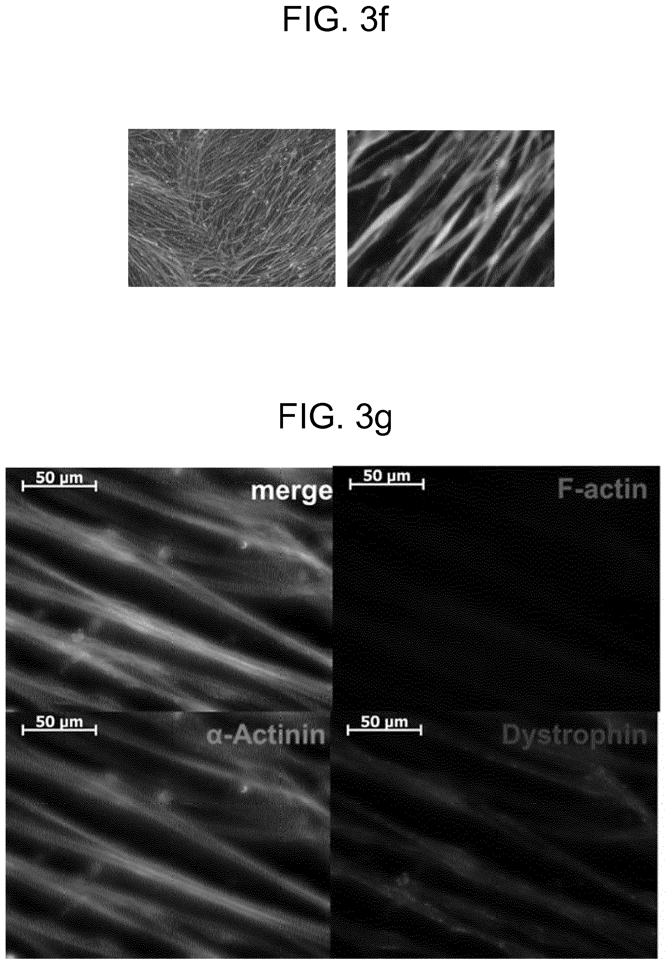

In order to study whether or not myotube cells could be induced from skeletal muscle precursor cells obtained from the frozen cell preparation, as mentioned above, the frozen cell preparation was quickly thawed on a water bath of 37°C, then plated over DMEM (high glucose) medium supplemented with 10 µg/mL FGF2, 10 µM FSK, 10 µM Y-27632, 5% KSR, 1% AA and 1% ITS-G in a Matrigel-coated plate, and cultured for 2 days. The medium was replaced with DMEM (high glucose) medium supplemented with 10 µM FSK, 10 ng/mL FGF2, 1% AA, 5% KSR and 1% ITS-G. The cells were then cultured for 2 weeks with the medium replaced every 2 to 3 days. The expression of human skeletal muscle cell markers in the cells thus cultured for 2 weeks was detected by fluorescent immunostaining. The fluorescent immunostaining employed an antibody recognizing all myosin heavy chain subtypes as skeletal muscle markers (MYH clone MF20; indicated by green in the drawing) and an antibody against desmin (indicated by red in the drawing) confirmed to be expressed from the initial stage of differentiation from satellite cells. The nuclei were further stained with DAPI (indicated by blue in the drawing). As a result, the small spherical form of the cells at the time of plating was changed to a long thin form, as shown in Figure 3f, in association with differentiation, and each cell was further found to have a plurality of nuclei. This demonstrated that myotube cells can be induced from skeletal muscle precursor cells obtained from the frozen cell preparation.

### <3-5. Differentiation of myotube cells into skeletal muscle cells>

The frozen cell preparation was quickly thawed on a water bath of 37°C, then plated over DMEM (high glucose) medium supplemented with 10 µM Y-27632, 10 µM FSK, 50 ng/mL FGF1, 5% KSR, 1% AA and 1% ITS-G in a Matrigel-coated plate, and cultured for 1 day. The cells were cultured for 2 weeks with the medium replaced with DMEM (high glucose) medium supplemented with 10 µM FSK, 50 ng/mL FGF1, 5% KSR, 1% AA and 1% ITS-G every 2 to 3 days to obtain myotube cells. In order to study whether or not differentiation into skeletal muscle cells could be induced, the myotube cells were further cultured for 2 weeks with the medium replaced with DMEM (high glucose) medium supplemented with 5% KSR, 1% AA and 1% ITS-G (free from a growth factor and FSK) every 2 to 3 days. The expression of skeletal muscle proteins in the cells was examined by the fluorescent immunostaining method 4 weeks after the thawing of the frozen cell preparation (on week 7 of differentiation). As a result, as shown in Figure 3g, it was able to be confirmed that: α-actinin (indicated by green in the drawing) expressed in the sarcomere of skeletal muscle was expressed in a striated pattern; dystrophin (indicated by red in the drawing) was expressed throughout the cells from near the cell membranes; and fibrous actin (F-actin; indicated by blue in the drawing) intracellularly bound with dystrophin resided in a fibrous pattern within the cells. These results demonstrated that the method of the present invention can induce skeletal muscle cells morphologically similar to natural skeletal muscle cells.

### <3-6. Differentiation of skeletal muscle precursor cells obtained from frozen cell preparation into myotube cells (study on type of growth factor)>

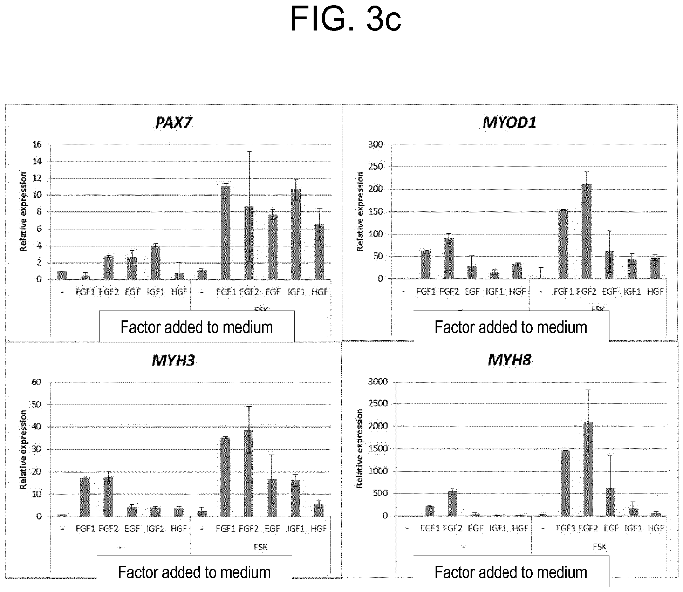

A culture supernatant was removed from the cells cultured in the same way as the method described in Example 2 on week 3 of differentiation. The cells were washed with phosphate-buffered saline, then dispersed by the addition of ACCUMAX (Innovative Cell Technologies, Inc.), and cryopreserved using Cellbanker 2 (Nippon Zenyaku Kogyo Co., Ltd.) to prepare a frozen cell preparation. In order to study the influence of the type of a growth factor to be added to a medium and the presence or absence of addition of FSK on the induction of further differentiation of skeletal muscle precursor cells obtained from the frozen cell preparation, the induction of differentiation was performed using media supplemented with these factors in the combinations shown in Figure 3c. Specifically, the frozen cell preparation was quickly thawed on a water bath of 37°C, then plated over DMEM (high glucose) medium supplemented with 10 µM Y-27632, 5% KSR, 1% AA and 1% ITS-G with or without the addition of a growth factor (50 ng/mL FGF1, 10 ng/mL FGF2, 50 ng/mL EGF, 50 ng/mL IGF1 or 50 ng/mL HGF) and 10 µM FSK according to the need in a Matrigel-coated plate, and cultured for 1 day. The cells thus cultured were cultured for 2 weeks with the medium replaced every 2 to 3 days with DMEM (high glucose) medium supplemented with 5% KSR, 1% AA and 1% ITS-G with or without the addition of a growth factor (50 ng/mL FGF1, 10 ng/mL FGF2, 50 ng/mL EGF, 50 ng/mL IGF1 or 50 ng/mL HGF) and 10 µM FSK according to the need.

Two weeks after the plating (on week 5 of differentiation), the cells were recovered, and the gene expression levels of a skeletal muscle precursor cell marker (PAX7) and skeletal muscle markers (MYH3, MYH8 and MYOD1) were measured in the same way as the method described in Example 1. As a result, as shown in Figure 3c, the cells recovered 2 weeks after the plating (corresponding to week 5 of differentiation) were found to express PAX7 and MYH3, MYH8 and MYOD1. In Figure 3c, a relative amount of change was calculated with a growth factor non-addition group as a reference sample. The culture in the medium containing each growth factor induced the expression of PAX7 and three skeletal muscle markers and synergistically increased the expression levels of these markers by the addition of FSK into the medium together with the growth factor.

### <3-7. Differentiation of skeletal muscle precursor cells into skeletal muscle cells>

A culture supernatant was removed from the cells on week 3 of differentiation. The cells were washed with phosphate-buffered saline, then dispersed in ACCUMAX (Innovative Cell Technologies, Inc.), then plated, without being cryopreserved, over DMEM (high glucose) containing 10 µM FSK, 10 ng/mL FGF2, 5% KSR, 1% AA, 1% ITS-G and 10 µM Y-27632 (added only at the time of plating) in another Matrigel-coated plate, and then cultured for 2 weeks with the medium replaced with DMEM (high glucose) medium supplemented with 10 µM FSK, 10 ng/mL FGF2, 5% KSR, 1% AA and 1% ITS-G every 2 to 3 days. After the culture, the cells 2 weeks to 4 weeks after dispersion and plating (on weeks 5 to 7 of differentiation) were further cultured in DMEM (high glucose) medium supplemented with 5% KSR, 1% AA and 1% ITS-G (free from a growth factor and FSK).

The cells on week 7 of differentiation were immunostained and morphologically observed. The immunostaining was performed by the following method: the cells were fixed in 4% paraformaldehyde, then lysed with 0.3% Triton X100, then reacted with a primary antibody solution containing an anti-α-sarcomeric actinin antibody (Sigma-Aldrich Co. LLC), an anti-dystrophin antibody (Abcam plc.) or an anti-β-dystroglycan antibody (Santa Cruz Biotechnology, Inc.), and further reacted with an Alexa Fluor 488 or 568-labeled secondary antibody solution. F-actin was stained with Alexa Fluor 350-labeled phalloidin (Thermo Fisher Scientific Inc.), and the nuclei were stained with DAPI (Wako Pure Chemical Industries, Ltd.).

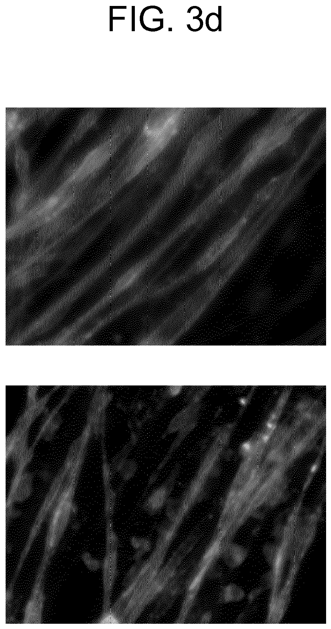

The expression of skeletal muscle marker proteins in the cells was examined by the fluorescent immunostaining method. As a result, as shown in Figure 3d (upper diagram), the cells exhibited a long thin form where α-sarcomeric actinin (indicated by red in the drawing) was intracellularly localized in a striated pattern, and dystrophin (indicated by green in the drawing) was observed on the cell membranes or throughout the cells. As also shown in Figure 3d (lower diagram), it was found that: a membrane protein β-dystroglycan (indicated by green in the drawing), which forms a complex with dystrophin (indicated by red in the drawing), exhibited similar localization; and F-actin (indicated by blue in the drawing) intracellularly bound with dystrophin was abundant within the cells (Figure 3d (lower diagram)). These results demonstrated that skeletal muscle cells induced by the method of the present invention are morphologically similar to natural skeletal muscle cells.

### Example 4: Purification of skeletal muscle precursor cells induced from iPS cells

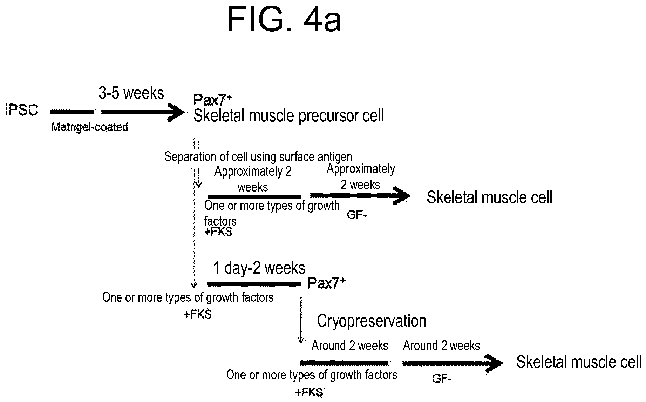

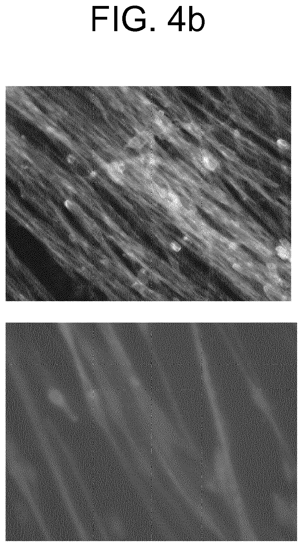

On week 3 of differentiation, CD56-positive cells were purified from the cells dispersed in ACCUMAX using a phycoerythrin (PE)-labeled antibody against CD56 (Miltenyi Biotec) and magnetic beads (EasySep(TM) Human PE Positive Selection Kit (StemCell Technologies Inc.)). In order to study whether or not skeletal muscle precursor cells could be induced from the purified CD56-positive cells, the purified CD56-positive cells were plated over a Matrigel-coated plate and cultured for 2 weeks with the medium replaced with DMEM (high glucose) medium supplemented with 10 ng/mL FGF2, 10 µM FSK, 50 KSR, 1% AA and 1% ITS-G every 2 to 3 days. The cells thus cultured were further cultured for 2 weeks with the medium replaced with DMEM (high glucose) medium supplemented with 5% KSR, 1% AA and 1% ITS-G (free from a growth factor and FSK) every 2 to 3 days. Figure 4a summarizes methods for purifying skeletal muscle precursor cells induced from human iPS cells and inducing differentiation of the purified skeletal muscle precursor cells into skeletal muscle, or the cryopreservation of the purified skeletal muscle precursor cells. As a result, as shown in Figure 4b, the cells thus cultured exhibited a long thin form where dystrophin (indicated by green in the drawing), β-dystroglycan (indicated by red in the drawing), and F-actin (indicated by blue in the drawing) were observed throughout the cells (Figure 4b, upper diagram). α-Sarcomeric actinin expressed on sarcomere Z membranes were localized in the longitudinal direction within long cells, and the nuclei expressed a skeletal muscle-specific transcriptional factor MYOD which developed red color (Figure 4b, lower diagram). This demonstrated that skeletal muscle cells can also be induced from the skeletal muscle precursor cells purified with CD56.

### <Freezing of purified cells and differentiation of cells obtained from frozen cell preparation into skeletal muscle cells>

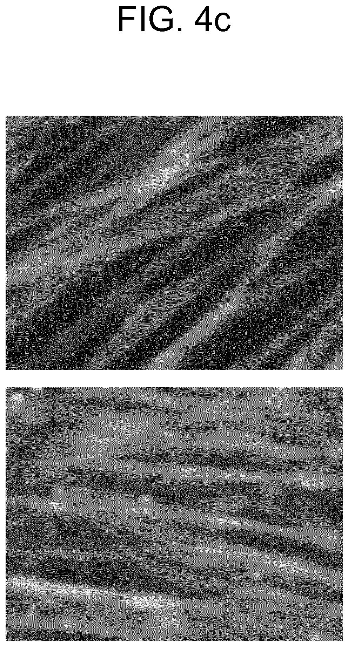

In order to study whether or not the skeletal muscle precursor cells purified with CD56 could also be cryopreserved, the following study was conducted: the cells purified with CD56 were plated over DMEM (high glucose) medium supplemented with 10 µM FSK, 10 ng/mL FGF2, 5% KSR, 1% AA and 1% ITS-G in a Matrigel-coated plate, and cultured for 2 days. The cells thus cultured were dispersed in ACCUMAX. The obtained cells were cryopreserved with Cellbanker 2 (Nippon Zenyaku Kogyo Co., Ltd.) to prepare a frozen cell preparation. The frozen cell preparation was quickly thawed on a water bath of 37°C, then plated over DMEM (high glucose) medium supplemented with 10 µM Y-27632, 10 ng/mL FGF2, 10 µM FSK, 5% KSR, 1% AA and 1% ITS-G in a Matrigel-coated plate, and cultured for 1 day. The cells thus cultured were further cultured for 2 weeks with the medium replaced with DMEM (high glucose) medium supplemented with 10 ng/mL FGF2, 10 µM FSK, 5%KSR, 1% AA and 1% ITS-G every 2 to 3 days. Then, the cells were further cultured for 2 weeks with the medium replaced with DMEM (high glucose) medium supplemented with 5% KSR, 1% ITS-G and 1% AA (free from a growth factor and FSK) every 2 to 3 days. The expression of dystrophin, β-dystroglycan and MYOD in the cultured cells was observed by the fluorescent immunostaining method. As a result, as shown in Figure 4c, it was found that: dystrophin (indicated by green in the drawing) resided on the cell membranes; and F-actin (indicated by blue in the drawing) resided within the cells (Figure 4c, upper diagram). It was also found that: α-sarcomeric actinin (indicated by green in the drawing) was expressed in a striated pattern within long cells; and one fiber contained a plurality of MYOD-positive nuclei (indicated by red in the drawing) (Figure 4c, lower diagram). This demonstrated that skeletal muscle cells can also be induced from the frozen cell preparation produced from the purified cells. These results demonstrated that skeletal muscle cells can also be induced from the frozen cell preparation produced from the cells purified with CD56.

### Example 5: Study on culture conditions

### <5-1: Reference culture conditions>

According to Reference Example 1, 253G1 cells were plated over a Matrigel-coated culture vessel. Two days thereafter, the E8 medium was replaced with B-27 medium supplemented with 5 µM CHIR and 1% AA to start differentiation (the starting day of differentiation was defined as day 0 of differentiation).

On day 2 of differentiation, the medium was replaced with B-27 medium supplemented with 1% AA, 10 µM SB and 0.5 µM DM. On day 4 of differentiation, the medium was further replaced with B-27 medium supplemented with 1% AA and 10 µM FSK. The culture was continued until day 7 of differentiation.

On day 7 of differentiation, the medium was replaced with DMEM (high glucose) medium supplemented with 10 µM FSK, 5% StemSure Serum Replacement, 1% AA and 1% ITS-G. The culture was continued with the medium replaced every 2 to 3 days.

The cells (skeletal muscle precursor cells) on week 3 of differentiation were cryopreserved in the same way as the method described in Example 3. The cryopreserved cells were thawed, plated, and cultured for 2 days in the same way as the method described in Example 3. Then, the medium was replaced with DMEM (high glucose) medium supplemented with 10 µg/mL FGF2, 10 µM FSK, 1% AA, 5% StemSure Serum Replacement and 1% ITS-G. The culture was continued for 2 weeks with the medium replaced every 2 to 3 days.

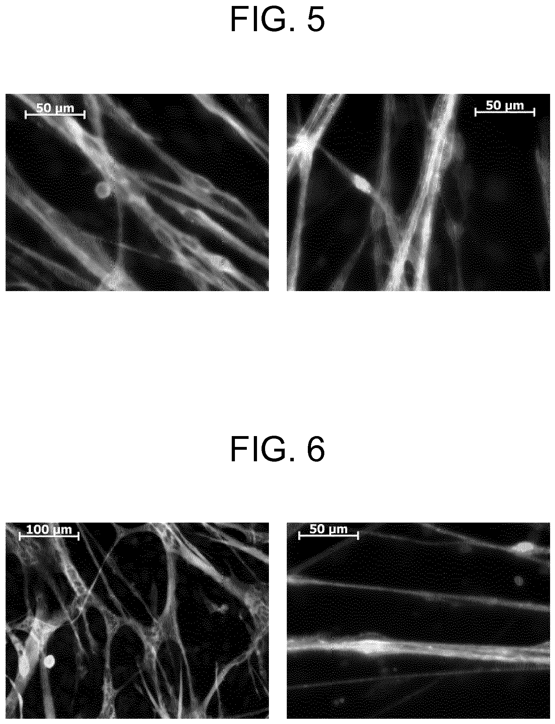

The cells thus cultured for 2 weeks were fluorescently immunostained using an antibody recognizing myosin heavy chains (R&D Systems, Inc., clone No.: MF20), an antibody recognizing desmin (Abcam plc.) and Hoechst 33342 for nuclear staining (Dojindo Laboratories). As a result, multinuclear myotube cells costained with both the antibodies were confirmed (Figure 5, left diagram).

On week 2 of culture, the medium was replaced with growth factor- and FSK-free DMEM (low glucose) medium supplemented with 1% AA, 5% StemSure Serum Replacement and 1% ITS-G, and the culture was continued with the medium replaced. On week 4 of culture, the cells were fluorescently immunostained using an antibody recognizing α-actinin (Sigma-Aldrich Co. LLC), an antibody recognizing dystrophin (Abcam plc.) and Hoechst 33342. As a result, multinuclear skeletal muscle cells were stained (Figure 5, right diagram).

Various changes in conditions were studied with reference to the experiment described above. The frozen cells on weeks 2 and 4 of culture were similarly fluorescently immunostained and evaluated.

### <5-2: Study on coating>

Cells were obtained in the same way as the method described in the preceding section "5-1" except that a culture vessel coated with fibronectin (Sigma-Aldrich Co. LLC) instead of Matrigel was used. After thawing, myotube cells (Figure 6, left diagram) and skeletal muscle cells (Figure 6, right diagram) were confirmed on weeks 2 and 4, respectively, of culture.

### <5-3: Study on reagent to be added - 1>

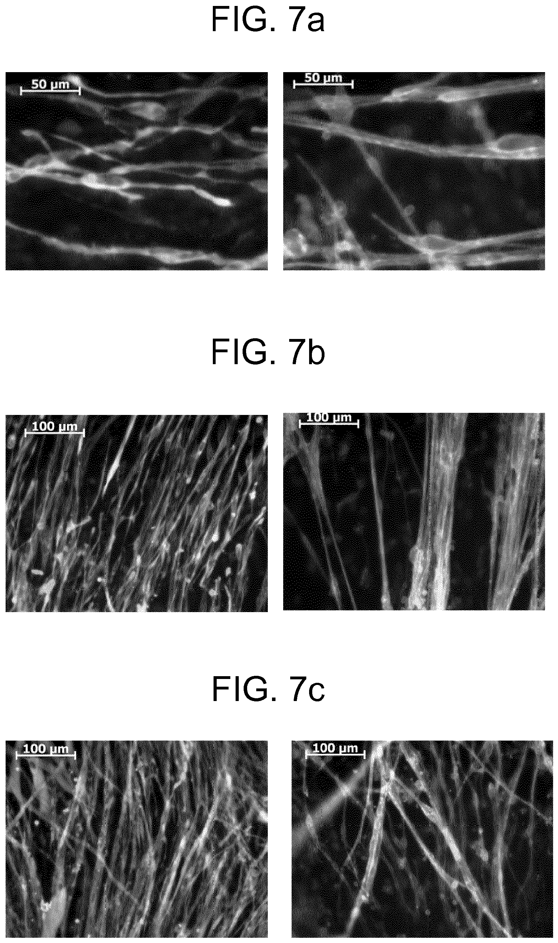

In the same way as in the preceding section "5-2", it was possible to use 1 µM A83-01 instead of SB (Figure 7a), and it was possible to use 1 µM A83-01 and 0.1 µM LDN193189 instead of SB and DM (Figure 7b).

In the same way as in the preceding section "5-2", it was possible to use 50 µM dbcAMP instead of FSK (Figure 7c).

### <5-4: Study on reagent to be added - 2>

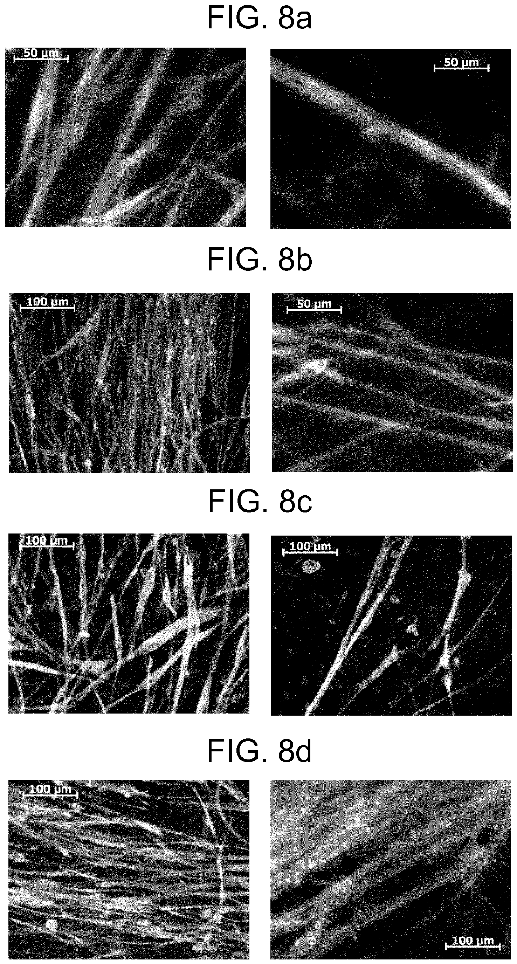

In the same way as in the preceding section "5-1", it was possible to use 0.5 µM BIO (Figure 8a) or 10 µM SB216763 (Figure 8b) instead of CHIR.

In the same way as in the preceding section "5-1", it was possible to start differentiation in a medium supplemented with 0.5 µM BIO instead of CHIR and to use 1 µM A83-01 instead of SB on day 2 of differentiation (Figure 8c), and it was possible to use 1 µM A83-01 and 0.1 µM LDN193189 instead of SB and DM (Figure 8d).

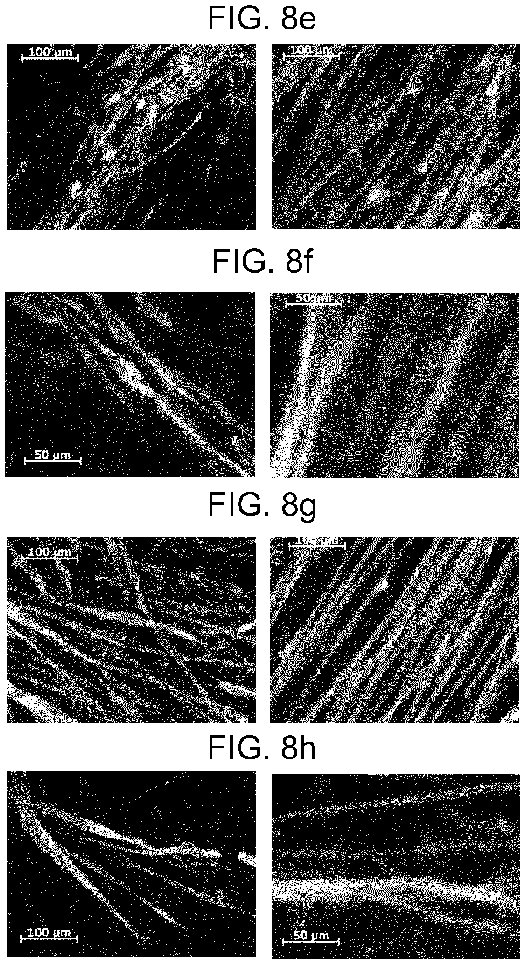

In the same way as in the preceding section "5-1", it was possible to start differentiation using 10 µM SB216763 instead of CHIR and to use 1 µM A83-01 instead of SB on day 2 of differentiation (Figure 8e).

In the same way as in the preceding section "5-1", it was possible to start differentiation using 10 µM SB216763 instead of CHIR, to replace the medium with a medium supplemented with SB and DM on day 2 of differentiation, and to use 50 µM dbcAMP instead of FSK from day 4 of differentiation (Figure 8f).

In the same way as in the preceding section "5-1", it was possible to use 1 µM A83-01 instead of SB on day 2 of differentiation (Figure 8g), and it was possible to use 1 µM A83-01 and 0.1 µM LDN193189 instead of SB and DM (Figure 8h).

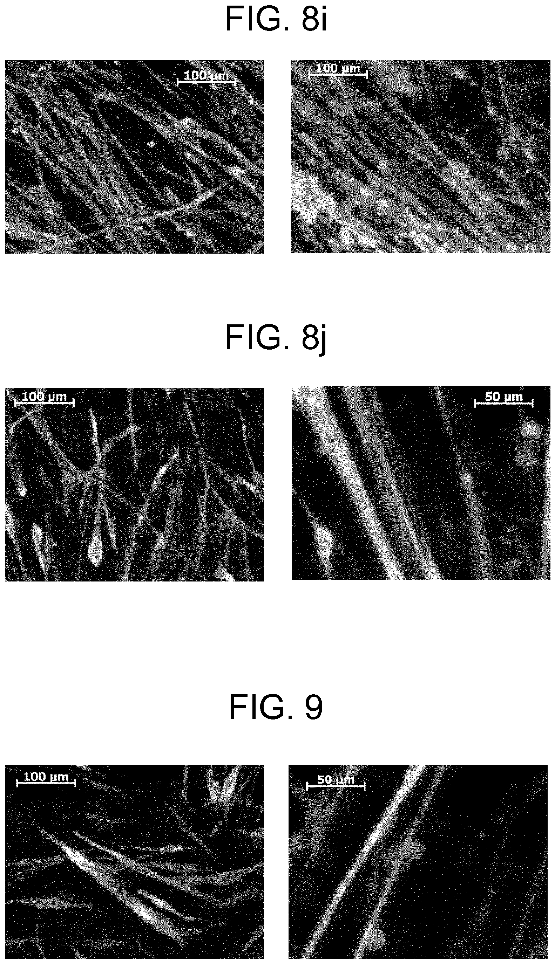

In the same way as in the preceding section "5-1", it was possible to induce differentiation into skeletal muscle precursor cells both in the case of adding 50 µM dbcAMP instead of FSK from day 4 of differentiation (Figure 8i) and in the case of not adding FSK from day 4 of differentiation (Figure 8j).

### <5-5: Study on cell line>

In the same way as in the preceding section "5-1", it was also possible to induce differentiation into skeletal muscle precursor cells in the case of using 201B7 cells instead of 253G1 cells (Figure 9).

The cell line and culture conditions of each drawing are summarized in Table 1.

**[Table 1]**

| No. of figure | Cell line | Conditions | | | |
|---|---|---|---|---|---|
| | | Plating Adhesion substrate | Reagent added on day 0 | Reagent added on day 2 | Reagent added on day 4 and day 7 to week 3 |
| No. of figure | 253G1 | Matrigel | CHIR99021 | SB+DM | FSK |
| Figure 6 | 253G1 | Fibronectin | CHIR99021 | SB+DM | FSK |
| Figure 7a | 253G1 | Fibronectin | CHIR99021 | A+DM | FSK |
| Figure 7b | 253G1 | Fibronectin | CHIR99021 | A+LDN | FSK |
| Figure 7c | 253G1 | Fibronectin | CHIR99021 | SB+DM | dbcAMP |
| Figure 8a | 253G1 | Matrigel | BIO | SB+DM | FSK |
| Figure 8b | 253G1 | Matrigel | SB216763 | SB+DM | FSK |
| Figure 8c | 253G1 | Matrigel | BIO | A+DM | FSK |
| Figure 8d | 253G1 | Matrigel | BIO | A+LDN | FSK |
| Figure 8e | 253G1 | Matrigel | SB216763 | A+DM | FSK |
| Figure 8f | 253G1 | Matrigel | SB216763 | SB+DM | dbcAMP |
| Figure 8g | 253G1 | Matrigel | CHIR99021 | A+DM | FSK |
| Figure 8h | 253G1 | Matrigel | CHIR99021 | A+LDN | FSK |
| Figure 8i | 253G1 | Matrigel | CHIR99021 | SB+DM | dbcAMP |
| Figure 8j | 253G1 | Matrigel | CHIR99021 | SB+DM | - |
| Figure 9 | 201B7 | Matrigel | CHIR99021 | SB+DM | FSK |

| | | | | | |
|---|---|---|---|---|---|
| CNIR:CHIR99021, SB:SB431542, DM:Dorsomorphin, A:A83-01, LDN:LDN193189 | | | | | |

### Test Example 1: Purities before and after purification operation

### 1. Measurement of purity of skeletal muscle precursor cells in cell preparation by flow cytometry

Two types of skeletal muscle precursor cell markers were stained, and the proportion of cells stained with both the antibodies (copositive) was calculated as a purity by flow cytometry.

### (1) Purity before purification operation

The cells cultured in the same way as the method described in Example 2 were dispersed in ACCUTASE on week 3 of differentiation. The dispersed cells were stained with an anti-CD56 antibody (Miltenyi Biotec) labeled with a fluorescent dye PE or an anti-CD29 antibody (Miltenyi Biotec) labeled with PE. Subsequently, each cell was fixed using Transcription Factor Buffer Kit (BD) and stained with an anti-Pax7 antibody (Novus Biologicals, LLC) labeled with a fluorescent dye Alexa Fluoro 488. For a control, the dispersed cells were stained with an isotype control antibody (BD) labeled with PE, then fixed using Transcription Factor Buffer Kit (BD), and stained with an isotype control antibody (Novus Biologicals, LLC) labeled with Alexa Fluoro 488. The proportion of cells stained with the fluorescent dyes was measured using FACSAria II (BD).

### (2) Purity after purification operation

The cells were stained with a PE-labeled anti-CD56 antibody or a PE-labeled anti-CD29 antibody by the method described in Example 4 or Test Example 1, and purified by the method described in Example 4. Subsequently, the cells were fixed and stained with an Alexa Fluoro 488-labeled anti-Pax7 antibody by the method described in Test Example 1. The purity was calculated by measurement using FACSAria II.

### 2. Results

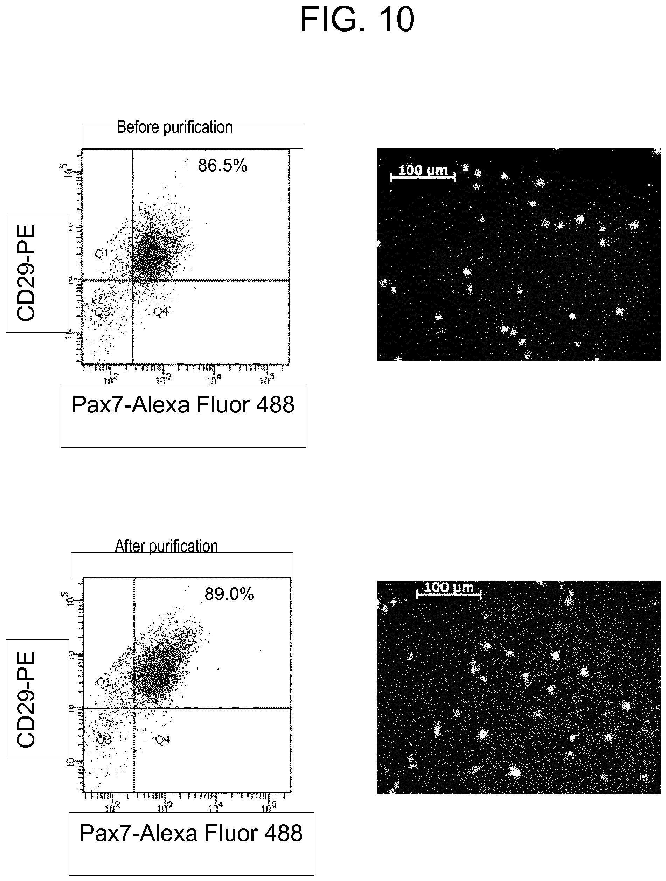

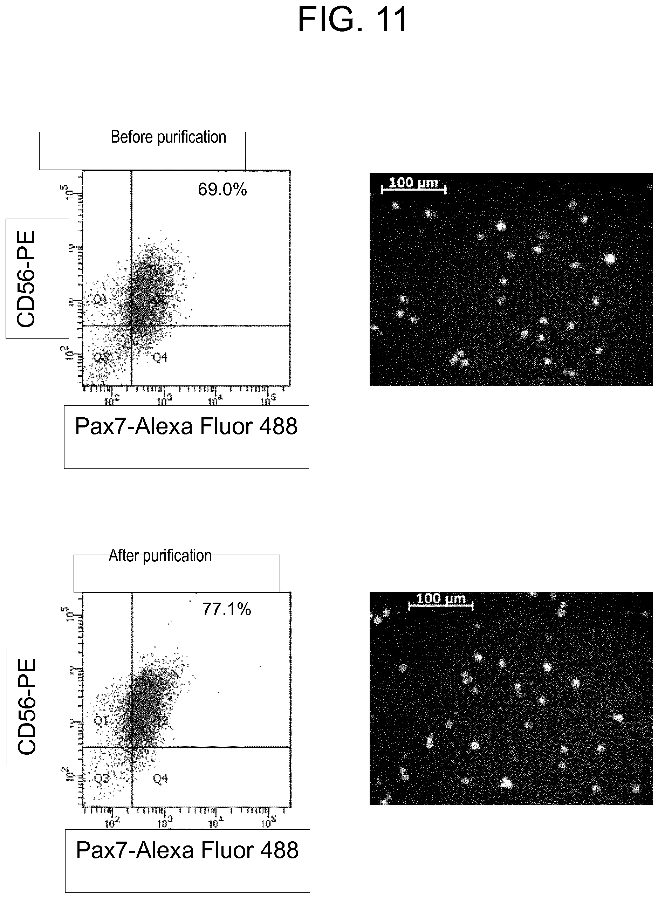

Figures 10 and 11 show the results of FACS and the results of observation under a fluorescence microscope. The ordinate depicts the fluorescence intensity of PE (which indicates cells stained with the anti-CD29 antibody or the anti-CD56 antibody), and the abscissa depicts the fluorescence intensity of Alexa Fluoro 488 (which indicates cells stained with the anti-Pax7 antibody). The cells before purification (upper) had a CD29/Pax7 copositive rate of 86.5% and a CD56/Pax7 copositive rate of 69.0%. On the other hand, the cells after the purification operation (lower) had a CD29/Pax7 copositive rate of 89.0% and a CD29/Pax7 copositive rate of 77.1%. Thus, the purity was elevated as compared with that before the purification operation.

### Industrial Applicability

The methods of the present invention can provide a cryopreservable highly pure skeletal muscle precursor cell preparation derived from pluripotent stem cells, and rapidly and stably provide skeletal muscle cells through the use of this cell preparation. The resulting skeletal muscle cell preparation is useful as a cell drug and, in addition, is useful for achieving drug screening.

## Claims

1. A method for producing skeletal muscle precursor cells, comprising the steps of:
(1) culturing pluripotent stem cells in adhesion culture in a medium containing a GSK3β inhibitor for 1-4 days;
(2) culturing the cells obtained in the step (1) in adhesion culture in a medium containing an ALK inhibitor for 1-4 days; and
(3) culturing the cells obtained in the step (2) in adhesion culture in a medium containing a cAMP inducer for 9-26 days.

2. The method according to claim 1, wherein the medium in the step (2) contains an AMP kinase inhibitor.

3. The method according to claim 1 or 2, wherein the media in the steps (1) to (3) are free from a growth factor.

4. The method according to any one of claims 1 to 3, wherein the skeletal muscle precursor cells are positive for CD56 and/or PAX7.

5. The method according to any one of claims 1 to 4, wherein the skeletal muscle precursor cells are positive for CD29 and/or PAX7.

6. The method according to any one of claims 1 to 5, further comprising the step of purifying CD56-positive cells.

7. The method according to any one of claims 1 to 6, further comprising the step of purifying CD29-positive cells.

8. A method for producing myotube cells, comprising
(1) culturing pluripotent stem cells in adhesion culture in a medium containing a GSK3β inhibitor for 1-4 days;
(2) culturing the cells obtained in the step (1) in adhesion culture in a medium containing an ALK inhibitor for 1-4 days;
(3) culturing the cells obtained in the step (2) in adhesion culture in a medium containing a cAMP inducer for 9-26 days to obtain skeletal muscle precursor cells positive for CD56 and/or CD29 and/or PAX7, and optionally purifying CD56-positive cells and/or CD29-positive cells;
(4) culturing the cells obtained in step (3), or cells obtained by thawing a frozen cell preparation obtained from cells obtained in step (3), in adhesion culture in a medium containing a growth factor for 7-20 days to induce differentiation into myotube cells.

9. The method according to claim 8, wherein the medium containing a growth factor of step (4) further contains a cAMP inducer.

10. The method according to claim 8, wherein the growth factor of step (4) is any one or two or more members selected from the group consisting of FGF2, FGF1, HGF, EGF and IGF1.

11. A method for producing skeletal muscle cells, comprising the steps of:
(1) culturing pluripotent stem cells in adhesion culture in a medium containing a GSK3β inhibitor for 1-4 days;
(2) culturing the cells obtained in the step (1) in adhesion culture in a medium containing an ALK inhibitor for 1-4 days;
(3) culturing the cells obtained in the step (2) in adhesion culture in a medium containing a cAMP inducer for 9-26 days to obtain skeletal muscle precursor cells, and optionally purifying CD56-positive cells and/or CD29-positive cells;
(4) culturing skeletal muscle precursor cells obtained in step (3) and positive for CD56 and/or CD29, in adhesion culture in a medium containing a growth factor; and
(5) culturing the cells obtained in the step (4) in a medium free from a growth factor.

12. The method according to claim 11, wherein the growth factor contained in the medium in the step (4) is any one or two or more members selected from the group consisting of FGF2, FGF1, HGF, EGF and IGF1.

## Patentansprüche

1. Verfahren zur Herstellung von Skelettmuskel-Vorläuferzellen, umfassend die folgenden Schritte:
(1) Kultivieren pluripotenter Stammzellen in Adhäsionskultur in einem Medium, das einen GSK3ß-Inhibitor enthält, für 1-4 Tage,
(2) Kultivieren der in Schritt (1) erhaltenen Zellen in Adhäsionskultur in einem Medium, das einen ALK-Inhibitor enthält, für 1-4 Tage, und
(3) Kultivieren der in Schritt (2) erhaltenen Zellen in Adhäsionskultur in einem Medium, das einen cAMP-Induktor enthält, für 9-26 Tage.

2. Verfahren nach Anspruch 1, wobei das Medium in Schritt (2) einen AMP-Kinase-Inhibitor enthält.

3. Verfahren nach Anspruch 1 oder 2, wobei die Medien in den Schritten (1) bis (3) frei von einem Wachstumsfaktor sind.

4. Verfahren nach einem der Ansprüche 1 bis 3, wobei die Skelettmuskel-Vorläuferzellen positiv für CD56 und/oder PAX7 sind.

5. Verfahren nach einem der Ansprüche 1 bis 4, wobei die Skelettmuskel-Vorläuferzellen positiv für CD29 und/oder PAX7 sind.

6. Verfahren nach einem der Ansprüche 1 bis 5, umfassend ferner den Schritt Aufreinigen der CD56-positiven Zellen.

7. Verfahren nach einem der Ansprüche 1 bis 6, umfassend ferner den Schritt Aufreinigen der CD29-positiven Zellen.

8. Verfahren zur Herstellung von Myotubenzellen, umfassend
(1) Kultivieren pluripotenter Stammzellen in Adhäsionskultur in einem Medium, das einen GSK3ß-Inhibitor enthält, für 1-4 Tage,
(2) Kultivieren der in Schritt (1) erhaltenen Zellen in Adhäsionskultur in einem Medium, das einen ALK-Inhibitor enthält, für 1-4 Tage,
(3) Kultivieren der in Schritt (2) erhaltenen Zellen in Adhäsionskultur in einem Medium, das einen cAMP-Induktor enthält, für 9-26 Tage zur Gewinnung von Skelettmuskel-Vorläuferzellen, die positiv für CD56 und/oder CD29 und/oder PAX7 sind, und gegebenenfalls Aufreinigen der CD56-positiven Zellen und/oder der CD29-positiven Zellen,
(4) Kultivieren der in Schritt (3) gewonnenen Zellen oder von Zellen, die durch Auftauen eines gefrorenen Zellpräparats erhalten wurden, das aus den in Schritt (3) erhaltenen Zellen gewonnen wurde, in Adhäsionskultur in einem Medium, das einen Wachstumsfaktor enthält, für 7-20 Tage zum Induzieren der Differenzierung in Myotubenzellen.

9. Verfahren nach Anspruch 8, wobei das einen Wachstumsfaktor enthaltende Medium von Schritt (4) außerdem einen cAMP-Induktor enthält.

10. Verfahren nach Anspruch 8, wobei der Wachstumsfaktor von Schritt (4) ein beliebiges oder zwei oder mehrere aus der aus FGF2, FGF1, HGF, EGF und IGF1 bestehenden Gruppe ausgewählten Elemente ist.

11. Verfahren zur Herstellung von Skelettmuskelzellen, umfassend die folgenden Schritte:
(1) Kultivieren pluripotenter Stammzellen in Adhäsionskultur in einem Medium, das einen GSK3ß-Inhibitor enthält, für 1-4 Tage,
(2) Kultivieren der in Schritt (1) erhaltenen Zellen in Adhäsionskultur in einem Medium, das einen ALK-Inhibitor enthält, für 1-4 Tage,
(3) Kultivieren der in Schritt (2) erhaltenen Zellen in Adhäsionskultur in einem Medium, das einen cAMP-Induktor enthält, für 9-26 Tage zur Gewinnung von Skelettmuskel-Vorläuferzellen und gegebenenfalls Aufreinigen der CD56-positiven Zellen und/oder der CD29-positiven Zellen,
(4) Kultivieren der in Schritt (3) gewonnenen Skelettmuskel-Vorläuferzellen, die positiv für CD56 und/oder CD29 sind, in Adhäsionskultur in einem Medium, das einen Wachstumsfaktor enthält und
(5) Kultivieren der in Schritt (4) erhaltenen Zellen in einem Medium, das frei von einem Wachstumsfaktor ist.

12. Verfahren nach Anspruch 11, wobei der in dem Medium in Schritt (4) enthaltene Wachstumsfaktor ein beliebiges oder zwei oder mehrere aus der aus FGF2, FGF1, HGF, EGF und IGF1 bestehenden Gruppe ausgewählten Elemente ist.

## Revendications

1. Procédé de production de cellules précurseurs de muscles squelettiques, comprenant les étapes suivantes :
1) cultiver des cellules souches pluripotentes en culture adhérente dans un milieu contenant un inhibiteur de GSK3β, durant 1 à 4 jours,
2) cultiver les cellules obtenues dans l'étape (1) en culture adhérente dans un milieu contenant un inhibiteur d'ALK, durant 1 à 4 jours,
3) et cultiver les cellules obtenues dans l'étape (2) en culture adhérente dans un milieu contenant un inducteur d'AMPc, durant 9 à 26 jours.

2. Procédé conforme à la revendication 1, dans lequel le milieu utilisé dans l'étape (2) contient un inhibiteur d'AMP kinase.

3. Procédé conforme à la revendication 1 ou 2, dans lequel les milieux utilisés dans les étapes (1) à (3) ne contiennent pas de facteur de croissance.

4. Procédé conforme à l'une des revendications 1 à 3, dans lequel les cellules précurseurs de muscles squelettiques sont positives pour CD56 et/ou PAX7.

5. Procédé conforme à l'une des revendications 1 à 4, dans lequel les cellules précurseurs de muscles squelettiques sont positives pour CD29 et/ou PAX7.

6. Procédé conforme à l'une des revendications 1 à 5, qui comprend en outre une étape de purification des cellules CD56-positives.

7. Procédé conforme à l'une des revendications 1 à 6, qui comprend en outre une étape de purification des cellules CD29-positives.

8. Procédé de production de cellules myotubes, comprenant :
1) cultiver des cellules souches pluripotentes en culture adhérente dans un milieu contenant un inhibiteur de GSK3β, durant 1 à 4 jours,
2) cultiver les cellules obtenues dans l'étape (1) en culture adhérente dans un milieu contenant un inhibiteur d'ALK, durant 1 à 4 jours,
3) cultiver les cellules obtenues dans l'étape (2) en culture adhérente dans un milieu contenant un inducteur d'AMPc, durant 9 à 26 jours, pour obtenir des cellules précurseurs de muscles squelettiques positives pour CD56 et/ou CD29 et/ou PAX7, et en option, purifier les cellules CD56-positives et/ou les cellules CD29-positives,
4) et cultiver les cellules obtenues dans l'étape (3), ou des cellules obtenues par décongélation d'une préparation de cellules congelées obtenue à partir des cellules obtenues dans l'étape (3), en culture adhérente dans un milieu contenant un facteur de croissance, durant 7 à 20 jours, pour induire leur différenciation en cellules myotubes.

9. Procédé conforme à la revendication 8, dans lequel le milieu contenant un facteur de croissance, utilisé dans l'étape (4), contient en outre un inducteur d'AMPc.

10. Procédé conforme à la revendication 8, dans lequel le facteur de croissance de l'étape (4) est choisi, au nombre d'un ou deux ou plus, parmi les éléments de l'ensemble formé par les FGF2, FGF1, HGF, EGF et IGF1.

11. Procédé de production de cellules de muscles squelettiques, comprenant les étapes suivantes :
1) cultiver des cellules souches pluripotentes en culture adhérente dans un milieu contenant un inhibiteur de GSK3β, durant 1 à 4 jours,
2) cultiver les cellules obtenues dans l'étape (1) en culture adhérente dans un milieu contenant un inhibiteur d'ALK, durant 1 à 4 jours,
3) cultiver les cellules obtenues dans l'étape (2) en culture adhérente dans un milieu contenant un inducteur d'AMPc, durant 9 à 26 jours, pour obtenir des cellules précurseurs de muscles squelettiques, et en option, purifier les cellules CD56-positives et/ou les cellules CD29-positives,
4) cultiver les cellules précurseurs de muscles squelettiques obtenues dans l'étape (3) et positives pour CD56 et/ou CD29, en culture adhérente dans un milieu contenant un facteur de croissance,
5) et cultiver les cellules obtenues dans l'étape (4) dans un milieu ne contenant pas de facteur de croissance.

12. Procédé conforme à la revendication 11, dans lequel le facteur de croissance de l'étape (4) est choisi, au nombre d'un ou deux ou plus, parmi les éléments de l'ensemble formé par les FGF2, FGF1, HGF, EGF et IGF1.
